(19) Europäisches Patentamt European Patent Office Office européen des brevets

(11) **EP 2 787 840 B1**

(12) **EUROPEAN PATENT SPECIFICATION**

(45) Date of publication and mention
of the grant of the patent:
**07.06.2017 Bulletin 2017/23**

(21) Application number: **12829157.2**

(22) Date of filing: **07.12.2012**

(51) Int Cl.:
*A23L 33/10* (2016.01)    *A23L 33/16* (2016.01)

(86) International application number:
**PCT/IB2012/002866**

(87) International publication number:
**WO 2013/084064 (13.06.2013 Gazette 2013/24)**

(54) **EXTRUDED LEGUME FOOD PRODUCTS CONTAINING YEAST AUTOLYSATE**

EXTRUDIERTE HÜLSENFRUCHTPRODUKTE MIT HEFEAUTOLYSAT

PRODUITS EXTRUDÉS À BASE DE LÉGUMINEUSES CONTENANT UN AUTOLYSAT DE LEVURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2011 US 201113316231**

(43) Date of publication of application:
**15.10.2014 Bulletin 2014/42**

(73) Proprietors:
• **Lesaffre et Compagnie**
  **75001 Paris (FR)**
• **The United States of America as represented by The Secretary of Agriculture**
  **Washington, DC 20250 (US)**

(72) Inventors:
• **BERRIOS, Jose De J.**
  **Albany, CA 94710 (US)**
• **JÜSTEN, Peter**
  **92500 Rueil-Malmaison (FR)**
• **CHEONG, Wei Hua Adeline**
  **Daly City, CA 94014 (US)**

(74) Representative: **Cabinet Plasseraud**
  **66 rue de la Chaussée d'Antin**
  **75440 Paris Cedex 09 (FR)**

(56) References cited:
  **WO-A2-2008/118585      US-A- 5 132 127**
  **US-A1- 2008 145 483      US-A1- 2008 248 180**

• **WESTERN REGIONAL RES CENTER: "VALUE-ADDED EXTRUDED FOOD PRODUCTS FORMULATED WITH NUTRITIONAL YEAST AND LEGUME PULSES", , 15 January 2010 (2010-01-15), pages 1-3, XP002699500, Retrieved from the Internet: URL:http://www.reeis.usda.gov/web/crisproj ectpages/0412648-value-added-extruded-food -products-formulated-with-nutritional-yeas t-and-legume-pulses.html [retrieved on 2013-06-25]**

**Description**

**BACKGROUND OF THE INVENTION**

Legumes include the pulses and other well-known plants that bear legume fruits including, but not limited, to soybean, lupins, groundnut (such as peanuts) and clover.

Pulses are annual leguminous crops yielding from one to twelve grains or seeds of variable size, shape and color within a pod, harvested solely for dry grain. In accordance with the Food and Agricultural Organization of the United Nations (FAO), 11 primary pulses are recognized: Dry beans, Dry broad beans, Dry peas, Chickpea, Dry cowpea, Pigeon pea, Lentil, Bambara groundnut, Vetch, Lupins, and Minor pulses (Lablab, hyacinth bean (*Lablab purpureus*), Jack bean (*Canavadia ensiformis*), sword bean (*Canavadia gladiata*), Winged bean (*Psophocarpus teragonolobus*), Velvet bean, cowitch (*Mucuna pruriens var. utilis*), Yam bean (*Pachyrrizus erosus*))*.

One disadvantage associated with the consumption of dry beans and other pulses, is their long cooking time needed to soften the beans to an edible texture. The loss in cooking quality is associated with the development of hardness in stored dry beans and recognized as the hard-to-cook (HTC) phenomenon. The HTC phenomenon is the result of multiple physiological-chemical mechanisms. High temperatures and high relative humidities accelerate the development of the HTC phenomenon in stored dry beans (Berrios et al., 1998; Berrios et al., 1999). Due to the long cooking time required for cotyledon softening, HTC beans result in increased energy utilization, inferior nutritional quality, and poor acceptance by consumers (Bressani et al., 1963). Efforts to increase the utilization of beans have employed a variety of scientific approaches and processing techniques such as germination, fermentation, dehulling, fractionation, autoclaving, roasting, canning, drum drying and most recently the use of extrusion cooking.

Extrusion is a technology that involves heating a food material and/or food ingredients to relatively high temperature under pressure until it melts, and then releasing it into the ambient atmosphere, causing it to expand and solidify. The resulting product is a shelf-stable convenience, ready-to-eat food. Extrusion cooking offers the advantages of versatile storage options, low production costs, energy efficiency and shorter cooking times (Harper 1981).

Fast cooking using extrusion technology, is an alternative to the long boiling and other traditional forms of cooking legumes.

Document US 2008/145483 concerns an extrusion process for producing a legume food product with expression ratio wherein said expression ratio is uniform. The uniform expansion ratio possessed by the extruded product provides a consistent texture and has application in a wide variety of food consumables, ranging from snacks to breakfast cereals.

Document of Western Regional Res Center: "Value-added extruded food products formulated with nutritional yeast and legume pulses" (retrieved from internet http://www.reeis.usda.gov/web/crisprojectpages/0412648-value-added-extruded-food-products-formulated-with-nutritional-yeast-and-legume-pulses.html) describes a project for investigating the use of extrusion technology for the development of value-added food products formulated with yeast and legume pulses. More precisely, the approach consists in processing various formulations containing yeast and legumes pulses as major ingredient using selected extrusion processing conditions for moisture, temperature and screw speeds for the development of value-added food.

**SUMMARY OF THE INVENTION**

An embodiment of the invention is a food product for use in the treatment of obesity or for use in decreasing retroperitoneal adipose accumulation, wherein said food product comprises extruded legumes having uniform expansion ratio values of 6 or greater fortified with yeast autolysate.

According to a further embodiment of the invention, the extruded legume - nutritional yeast fortified food product contains chromium.

**BRIEF DESCRIPTION OF THE DRAWINGS**

FIG. 1 is a surface plot of the diameter of the extrudate versus feed moisture and die temperature.
FIG. 2 is a surface plot of diameter of expansion ratio of the extrudate versus feed moisture and die temperature.
FIG. 3 is a surface plot of die pressure versus feed moisture and die temperature.
FIG. 4 is a graph of extrusion processing parameters on the proximate composition of extruded lentil flours.
FIG. 5 is a surface plot of water activity (Aw) versus feed moisture and die temperature.
FIG. 6 is a surface plot of in vitro protein digestibility (IVPD) versus feed moisture and die temperature.
FIG. 7 is a surface plot of lightness (L) versus feed moisture and die temperature.
FIG. 8 is a surface plot of color index (DE) versus feed moisture and die temperature.

FIG. 9 shows a surface plot of specific mechanical energy (SME) versus feed moisture and die temperature.

FIG. 10 is a photo of product shapes due to speed and angle of the cutter.

FIG. 11 is a graph of the effect of different starch sources on physical properties of lentil based extrudates.

FIG. 12 is a graph of the effect of screw speed on physical properties of lentil based extrudates.

FIG 13 is a graph of texture modifier agents incorporated into the lentil based extrudate.

FIG 14 is a graph of the rate of moisture loss by the lentil extrudate during toasting.

FIG 15 is a graph of the body weight of normal phenotype mice and Ob/Ob mice fed the experimental diets, with and without chromium addition. OA: Obese mice fed diet containing snacks in Table 1, 1st formula; OB: Obese mice fed diet containing snacks in Table 1, 2nd formula; OC: Obese mice fed diet containing snacks made from formulations indicated in Table 1, 2nd formula, containing chromium provided by the product LYNSIDE® Forte Cr2000.

FIG 16 is a graph of liver, mesentery adipose, epididymal adipose, retroperitoneal adipose, kidney weights in obese and normal mice fed diets with or without lentil based formulations containing chromium provided by the product LYNSIDE® Forte Cr2000 that comprises deactivated yeast and chromium.

DEFINITIONS

"Legumes" include pulses and other well known fruits that bear legume fruits, including, but not limited to soybean, lupins, groundnut (such as peanuts) and clover.

"Pulses" refers to annual leguminous crops yielding from one to twelve grains or seeds of variable size, shape and color within a pod, harvested solely for dry grain.

"Extrusion" is a high temperature, high pressure, short time process that transforms a variety of food raw materials and ingredients into modified intermediate and finish products.

"Melt" refers to the molten extrudate.

"Extrudate" refers to the product obtained through extrusion processing.

"Supercritical fluid extrusion" involves the coupling of supercritical fluids, particularly supercritical carbon dioxide, and extrusion processing.

"Co-extrusion processing" refers to a technique where of two or more different yet compatible foods and/or food ingredients are combined in an extrusion die. The food materials can come from two extruders or from an extruder and a pump. This process permits to make specific products; such as, products with two or more different textures or colors or flavors.

"Preconditioner" is an atmospheric or pressurized chamber in which raw granular foods and/or food ingredients are uniformly moistened or heated or both by contact with water or live steam before entering the extruder.

"Shelf stable" refers to the length of time that corresponds to a tolerable loss in quality of processed foods and other perishable items.

"Flashing" refers to the sudden evaporation of moisture that occurred at the extruder die end, when superheated water is suddenly exposed to ambient conditions.

"Expansion" relates to the physical transformation which is observed when pressurized, molten flour or melt is suddenly exposed to ambient conditions.

"Expansion Ratio" (ER), also referred as Sectional Expansion Index (SEI) and Radial Expansion Ratio (ER)radial, is expressed as the ratio between the cross-sectional area of the extrudate and the area of the die or as the ratio between the diameter of the extrudate and the die.

"Uniform expansion ratio" (UER) is defined as a condition in which the variation of the expansion ratio for randomly selected portions of an extruded rod is less than 20% of the mean expansion ratio, and variations in expansion ratios among different batches of the product produced with the same ingredients under the same process condition are less than 20% of the mean expansion ratio.

"Expansion Indexes" (EI) refers to the overall expansion of an extrudate that takes place in three dimensions i.e. cross sectional, longitudinal, and volumetric expansion. They are defined by the mathematical equation: VEI = SEI x LEI, where SEI is sectional expansion index, which characterized diametral expansion; LEI is longitudinal expansion index and VEI is volumetric or overall expansion index.

"Expansion parameters" include, but are not limited to, expansion and density.

"Density" by definition is mass per unit volume, expressed by the mathematical equation, $p = m/V$, where p is density, m is mass (kg), and V is volume ($m^3$).

"Product density" (D) refers to the measure of extrudate mass per unit of volume. The higher an extrudate density, the higher it's mass per volume.

"Water solubility index" (WSI) of an extruded product describes its solubility in water. The value is given as a percent on a dry weight basis, and is described by the mathematical equation, WSI = [(mass of dissolved solid in supernatant)/(mass of dry solids)] * 100

"Water absorption index" (WAI) of an extruded product describes its ability to absorb water. The value is given as a percent on a dry weight basis, and is described by the mathematical equation, WAI = [(mass of sediment)/(mass of dry solids)] * 100.

"Texture properties" of a food are that group of physical characteristics that arise from the structural elements of the food, are sensed by the feeling of touch, are related to the deformation, disintegration, and flow of the food under a force, and are measured objectively by functions of pressure, time, and distance. They include, but are not limited to, hardness, strength, mouthfeel and viscosity.

"Hardness" is a mechanical property of a material that characterizes its resistance to deformation. Therefore, hardness of an extruded product describes the amount of force needed to cause deformation.

"Strength" is most often used to describe a material's Yield Strength. Yield Strength is a mechanical property of a material that characterizes its resistance to deformation. Therefore, strength of an extruded product describes the amount of force needed to cause deformation.

"Lightness" is synonymous with brightness, which indicates the brightness or darkness of a color. A low lightness value indicates dark (black), while a high lightness value indicates bright (white).

"Hydration properties" include, but are not limited to, the water solubility index (WSI) and the water absorption index (WAI).

*"In vitro* protein digestibility" (IVPD) refers to observation made experimentally in the test-tube, as distinct from the natural living conditions, *in vivo.* IVPD is generally expressed as the percent of protein hydrolyzed by digestive proteolytic enzymes.

"Consumer tasting", referred also as "Hedonic scale", involves having potential consumers of a product evaluate various products and a small number of items on a ballot.

"Fortification" is the addition of nutrients in amounts significant enough to improve the nutritional value of the food. This may include addition of nutrients not normally associated with the food or addition to levels above that present in the unprocessed food.

"Glycemic Index" is a physiological measurement of carbohydrate quality, based on their immediate effects on blood-glucose levels. Glycemic index (GI) uses a scale of 0-100. Pure glucose serves as a reference point and is given a GI of 100. When Carbohydrates in foods are compared gram for gram, GI values of 55 or less are considered low GI foods, GI values from 55-69 are considered intermediate GI foods and those with GI 70 or more as high GI foods.

"Starch" refers to a carbohydrate polymer occurring in granular form certain plant species notably cereals, tubers, and pulses such as corn, wheat, rice, tapioca potato, pea etc. The polymer consists of linked anhydro-a-D-glucose units. It may have either a mainly linear structure (amylose) or a branched structure (amylopectin). The molecular weight of the constituent polymers, particularly amylose, varies between different starch sources. A single plant species may exist as hybrids with various proportions of amylose and amylopectin e.g. high amylose corn.

"Specialty Starch(es) or Starch Derivatives" a generic term for all products produced from native starch including modified starches and starch hydrolysis products. They are used to improve the processing, physical and chemical attributes and eating qualities of the food products and may also address nutritional needs, such as fiber in the diet.

"Decorticated" refers to the removal of the surface layer, bark, husk, membrane, or fibrous cover of a seed or grain.

"Particle size" refers to particles from flours and/or powders that have been sized to a particular dimension through standard size designed sieves or screens.

"Sieving" refers to a method for categorizing a flour's and/or powder's particle size by running them through standard size designed sieves or screens.

"Legume based flours and/or powders" refers to a mix containing legume flour and plant (legume, cereal, fruit and vegetables, tubers) material and/or their ingredients (starch, dietary fibers, pigments, flavor extracts, phytonutrients) and/or animal (dairy, other) material and/or their ingredients (protein, sugar, fat, flavor extracts, other) and/or microbial based ingredients (protein, dietary fibers, vitamins, minerals, other) and/or other conventional and non-conventional food grade ingredients (specialty starches, water and oil soluble vitamins, minerals, colors, flavors, other).

"Microbial fiber" refers to dietary fiber such as beta-1,3 glucan from nutritional yeast, which is grown specifically for its nutritive value.

"Nutritional Yeast" refers to a deactivated yeast or a yeast autolysate. Yeast autolysate contains all the starting material of the yeast cell with no separation of the soluble and insoluble fractions. Autolysis leads to the degradation of the yeast cell via its own endogenous enzymes. The autolysis of a yeast is performed under controlled conditions, especially in terms of temperature, pH and time, which may be readily determined by a person skilled in the art. The yeast autolysates that may be used in the food product according to the invention preferably contain from 9% to 18% in mass of yeast beta glucans.

## DETAILED DESCRIPTION OF THE INVENTION

The technical and practical constraints for the production of expanded legume based extrudates fall into two separate categories. The first category relates to the parameters of the extrusion process itself. These are controllable physical/structural factors such as moisture content and particle size of the extrusion feed, barrel temperature and pressure, and residence time, which have direct effect on the quality attributes of the extrudate, such as, expansion ratio, nutritional value, density, color, water solubility/absorption, and its textural properties. The second category pertains to the use of legume flours and/or powders and legume based flours and/or powders with functional food additives, which have direct effect on the healthful, sensorial and textural characteristics and appearance of the final extrudate. If the problems identified above could be properly addressed and resolved, pulses could be used in making highly nutritious, healthful and convenient ready-to-eat expanded extruded and co-extruded products.

An embodiment of the invention describes particular extrusion processing parameters applied to extruded legume flours and/or powders in a way that results in uniformly highly expanded, crispy, tasty and shelf-stable extrudates containing nutritional yeast. Nutritional yeast is an excellent source of protein, dietary fiber, vitamins and minerals. In addition to protein, dietary fiber, vitamins and minerals, it contains additional functional and beneficial components such as beta-1,3 glucan, trehalose, mannan and glutathione. Studies have show that these components have potential health benefits such as, improved immune response, reduction of cholesterol, and anti-cancer properties. Dietary fiber typically suggests a plant derived indigestible complex carbohydrate categorized as either water soluble or water insoluble; however, in accordance with an embodiment of the invention the indigestible carbohydrate may also be drawn from a microbial source, such as nutritional yeast. An embodiment of the invention is extruded pulse based snacks exhibiting a uniform expansion ratio value of 6 or greater fortified with nutritional yeast (NY) and optionally chromium. Yeast is present within a formulation with extruded legumes at a concentration of 2 - 20%, preferably 12 - 20% by weight. Chromium may be added at a concentration of 1.16 ppm - 6 ppm. Chromium is preferably chromium chloride or chromium picolinate. In one embodiment, the chromium chloride is incorporated into the food product in the form of a mixture of deactivated yeasts and chromium chloride (Cr-Y). The chromium chloride is mixed with the yeasts at the end of multiplication of the yeasts, for example before or after the step of drying the yeasts. A preferred embodiment is the incorporation of chromium chloride into the composition in the form of a mixture of deactivated yeasts and of chromium chloride, the chromium having been mixed with the yeasts before the drying step. Tables 1 - 9 show analysis and digestibility of these fortified snacks.

A further embodiment is the use of sieved formulations containing additives and/or food ingredients from plant and animal sources such as, but not limited to, cereals, legumes and dairy proteins; specialty starches; fruits, vegetables and grain-based fibers; microbial based ingredients such as protein, dietary fiber, vitamins and minerals; texture and flavor modifiers including emulsifiers; colors, water and oil soluble vitamins and minerals, and spices mixed at specific ratios, which result in commercial type, highly nutritious, convenient and appealing expanded snack and breakfast cereal-type products of different shapes and sizes.

Another embodiment is the use of the expanded extrudate as ingredients in, but not limited to, bakery products, confectionary products and nutraceuticals of different shapes and sizes. The shapes that can be obtained are consistent with those desired by one of skill in the art such as bars, rods, balls, curls and other shapes of varying sizes.

Legumes and/or powders and legume based flours thereof, which may be utilized, include but are not limited to dry beans (*Phaseolus spp.*), lentil (*Lens culinaris*), dry peas (*Pisum spp.*), chickpea or garbanzo (*Cicer arietinum*), soybean (*Glycine max*), broad bean (*Vicia faba*), dry cowpea or black-eyed pea *(Vigna sinensis; Dodichos sinensis)*, pigeon pea, cajan pea or Congo bean *(Cajanus cajan)*, bambara groundnut or earth pea *(Voandzeia subterranea)*, spring/common vetch *(Vicia sativa)*, lupins (*Lupinus spp.*), and minor pulses/pulses including: Lablab, hyacinth bean (*Lablab purpureus),* Jack bean *(Canavalia ensiformis*), sword bean (*Canavalia gladiata*), Winged bean (*Psophocarpus teragonolobus*), Velvet bean, cowitch (*Mucuna pruriens var. utilis*), Yam bean (*Pachyrrizus erosus*)*;* guar bean *(Cyamopsis tetragonoloba).*

Additionally, raw legume seeds may be utilized, wherein the seeds are singularly or in combination, whole, split or decorticated.

A further embodiment is the use of flavorings, coatings or colors The flavorings or coatings that may be utilized are inclusive of those routinely available to one of skill in the art, which include formulations of solids, pastes or liquids as well as natural or synthetic flavorings. The color of the extrudate may be enhanced or changed using natural or synthetic colors, readily available to one of skill in the art.

An additional embodiment is a food product comprising extruded legumes possessing uniform expansion ratio values of 6 or greater fortified with nutritional yeast for use as a medicament, particularly in the treatment of obesity and/or hypercholesterolemia. An additional embodiment is the use of extruded legumes possessing uniform expansion ratio values of 6 or greater fortified with nutritional yeast to increase protein digestibility of a food product and /or decrease the glycemic index of a food product and/or decrease retroperitoneal adipose accumulation.

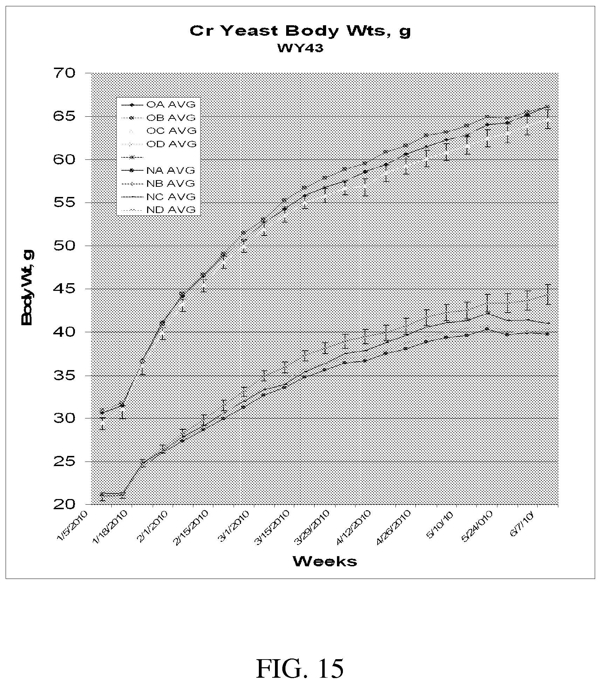

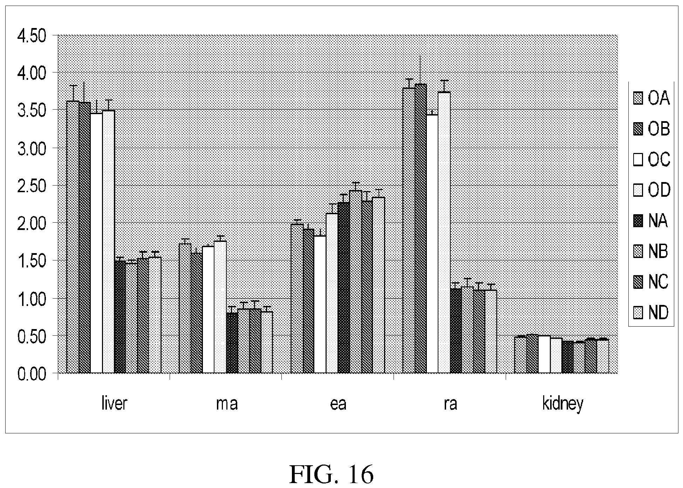

Another embodiment is the use of the to treat obesity, particularly the accumulation of retroperitoneal adipose. Consumption of a high glycemic index diet increases abdominal adiposity, Nut. Res., 30(2), pp. 141 - 150. The legume formulations containing a uniform expansion value ratio of 6 or greater, nutritional yeast and chromium present a snack composition with of low glycemic index and increased protein digestibility (see table 7 for protein digestibility). Genetically Obese mice (ob/ob) fed a standard mice diet with added Lentil-based extruded flour fortified with NY and up to 6 ppm Cr-Y, gained less weight than ob/ob mice on diets containing less Cr-Y or Cr from Cr Picolinate. Total length of the study: 21 weeks (see FIG. 15, OA: Obese mice fed diet containing snacks made from lentil-based formulations indicated in Table 1, 1st formula; OB: Obese mice fed diet containing snacks made from lentil-based formulations indicated in Table 1, 2nd formula, containing 1.25 ppm chromium provided by the product Lynside® Forte Cr2000 that comprises deactivated yeast and chromium; OC: Obese mice fed diet containing snacks made from lentil-based formulations indicated in Table 1, 2nd formula, containing 5.36 ppm chromium provided by the product Lynside® Forte Cr2000 that comprises deactivated yeast and chromium; OD: Obese mice fed diet containing 17.9 ppm chromium provided by chromium picolinate; NA: Normal mice fed diet containing snacks made from lentil-based formulations indicated in Table 1, 1st formula; NB: Normal mice fed diet containing snacks made from lentil-based formulations indicated in Table 1, 2nd formula, containing 1.25 ppm chromium provided by the product Lynside® Forte Cr2000 that comprises deactivated yeast and chromium; NC: Normal mice fed diet containing snacks made from lentil-based formulations indicated in Table 1, 2nd formula, containing 5.36 ppm chromium provided by the product Lynside® Forte Cr2000 that comprises deactivated yeast and chromium; ND: Normal mice fed diet containing 17.9 ppm chromium provided by chromium picolinate); and FIG. 16, OA: Obese mice fed diet containing snacks made from lentil-based formulations indicated in Table 1, 1st formula; OB: Obese mice fed diet containing snacks made from lentil-based formulations indicated in Table 1, 2nd formula, containing 1.25 ppm chromium provided by the product Lynside® Forte Cr2000 that comprises deactivated yeast and chromium; OC: Obese mice fed diet containing snacks made from lentil-based formulations indicated in Table 1, 2nd formula, containing 5.36 ppm chromium provided by the product Lynside® Forte Cr2000 that comprises deactivated yeast and chromium; OD: Obese mice fed diet containing 17.9 ppm chromium provided by chromium picolinate; NA: Normal mice fed diet containing snacks made from lentil-based formulations indicated in Table 1, 1st formula; NB: Normal mice fed diet containing snacks made from lentil-based formulations indicated in Table 1, 2nd formula, containing 1.25 ppm chromium provided by the product Lynside® Forte Cr2000 that comprises deactivated yeast and chromium; NC: Normal mice fed diet containing snacks made from lentil-based formulations indicated in Table 1, 2nd formula, containing 5.36 ppm chromium provided by the product Lynside® Forte Cr2000 that comprises deactivated yeast and chromium; ND: Normal mice fed diet containing 17.9 ppm chromium provided by chromium picolinate.

**Extrusion Process - Physical factors**

**Expansion**

Expansion relates to the physical transformation which is observed when molten flour (or "melt"), under high temperature and pressure, is suddenly exposed to ambient temperature and pressure. As the melt exits the extruder die, the sudden decrease in temperature and pressure causes the near-instantaneous expansion of the molten flour, which is also accompanied by extensive flushing or loss of moisture from the extruded product. The expansion of the extrudate, is one of the most important characteristics of interest for the snack food industry. (Mercier et al, 1989). There is limited information about expansion characteristics of legumes, since there is a conception that legumes' flours do not expand well. For this reason, legume flours and/or powders have not been used to produce expanded snacks and this type of products are made exclusively from mayor cereal grains (eg., corn, wheat and rice) and their starch-based flours were values greater than 20 have been obtained (Colonna et al., 1989; Meuser et al., 1894; Barret and Kaletunc, 1998). Soy protein with added starch has also been used for this purpose, but mainly for the fabrication of pet foods. Expansion is directly related to the moisture content of the feed, die temperature and pressure. Moreover, the particle size of the feed and extruder screw speed (Conway, 1971), as well as the presence of specific food ingredients in the formulation, have an important effect on the expansion and texture of the final extrudate. By properly selecting the above extrusion processing parameters and ingredients, it is possible to obtain desirable expansion, texture, nutritional value, color, and shelf stability in the finished product. Below is a discussion of how this is achieved by an embodiment of the invention.

According to an embodiment of the invention, as well as a highly expanded legume product, possessing expansion ratios of 6 or greater, the legume product is also uniform with regard to the expansion ratio. A uniform expansion ratio (UER) creates a uniform texture, which is an important and desired feature in food products, especially those products which may have additional coatings or flavorings added; moreover, a uniform expansion ratio ensures that the texture will be consistent within each batch processing of the extruded legume product. Table 1 demonstrates the uniform expansion ratio that can be achieved by an embodiment of the invention.

**Table 1. Lentil-based formulations fortified with yeast autolysate[1] and chromium[2] yeast**

| Ingredients | 1st formula | 2nd formula |
|---|---|---|
| **SRCL (*Split Red Chief Lentil*)** | **56%** | **56%** |

(continued)

| Ingredients | 1st formula | 2nd formula |
|---|---|---|
| Yeast autolysate | 12% | 12% |
| Chromium | 0% | 1.25 - 5.36 ppm |
| Starch | 20% | 20% |
| Fibers (*apple and wheat bran*) | 5% | 5% |
| Salt | 2% | 2% |
| Sugar (saccharose) | 5% | 5% |

[1]The yeast autolysate is the product Lynside® Nutri Snack Plus.
[2]The chromium is provided by the product Lynside® Forte Cr2000 that comprises deactivated yeast and chromium.

**Table 2. Physical Analyses Summary, Products**

| Description | Dia, mm | ER | BD,meas, g/cc |
|---|---|---|---|
| Formula ctrl | 13.71[a] | 15.43[a] | 0.0688[f] |
| 4%,140C | 11.50[c] | 10.84[c] | 0.1165[b] |
| 8%,140C | 11.40[c] | 10.64[c] | 0.1014[c] |
| 12%,140C | 10.61[c,d] | 9.23[d] | 0.1298[a] |
| 16%,140C | 11.69 | 11.18 | 0.1134 |
| 20%,140C | 11.21 | 10.30 | 0.1114 |
| 4%,160C | 11.76[c] | 11.32[b,c] | 0.0907[d] |
| 8%,160C | 11.80 | 11.40 | 0.0850 |
| 12%,160C | 12.45[b] | 12.76[b] | 0.0831[e] |
| 16%,160C | 11.49[c] | 10.80[c] | 0.0940[d] |
| 20%,160C | 11.50[c] | 10.86[c] | 0.0934[d] |
| SRCL ctrl | 16.47 | 22.75 | 0.1345 |

Values represent mean of three replicate analyses.
Means within a column followed by different letters (superscripts: a, b, c, d, e, f) are significantly different ($p < 0.05$).
Formula ctrl: contained all the ingredients indicated in Table 1, except the yeast autolysate and chromium.
SRCL ctrl : contained only SRCL.
x%: means x% of yeast autolysate.
xC: means x of degree Celsius at the extruded die.

**Table 3. Chemical Analyses, Products**

| Description | WAI | WSI | $A_w$ |
|---|---|---|---|
| Formula ctrl | 401.47[c] | 0.4238[a] | 0.3330[d] |
| 4%,140C | 465.26[a,b] | 0.4218[a] | 0.3817[b] |
| 8%,140C | 445.01[b] | 0.4192[a] | 0.3853[a] |
| 12%,140C | 454.36[b] | 0.4085[a] | 0.3927[a] |
| 4%,160C | 488.45[a] | 0.4091[a] | 0.3773[b] |
| 12%,160C | 458.21[b] | 0.3879[b] | 0.3663[c] |
| 16%,160C | 480.30[a,b] | 0.3684[b] | 0.3887[a] |

(continued)

| Description | WAI | WSI | $A_w$ |
|---|---|---|---|
| SRCL ctrl | 528.94 | 0.4235 | 0.4890 |

Values represent mean of three replicate analyses.
Means within a column followed by different letters (superscripts: a, b) are significantly different (p<0.05).
Formula ctrl: contained all the ingredients indicated in Table 1, except the yeast autolysate and chromium. SRCL ctrl : contained only SRCL.
x%: means x% of yeast autolysate.
xC: means x of degree Celsius at the extruded die.

**Table 4. Proximate Analysis Summary, Products**

| Description | Moisture, % | Fat, % | Ash, % | Protein, % | CHO, % |
|---|---|---|---|---|---|
| Formula ctrl | 8.84[a] | 0.38[b] | 1.92[e] | 22.10[d] | 65.90[a] |
| 4%,140C | 7.81[c] | 0.61[a] | 3.72[d] | 23.60[c] | 63.78[b] |
| 8%,140C | 7.75[c] | 0.60[a] | 3.89[c] | 25.21[b] | 61.90[c] |
| 12%,140C | 7.84[b,c] | 0.64[a] | 4.27[a] | 25.00[b] | 61.55[c] |
| 4%,160C | 7.60[c] | 0.65[a] | 3.81[d] | 24.18[c] | 63.15[b] |
| 12%,160C | 7.67[c] | 0.73[a] | 4.02[c] | 25.34[b] | 61.61[c] |
| 16%,160C | 8.02[b] | 0.67[a] | 4.11[b] | 26.13[a] | 60.51[d] |

Values represent mean of three replicate analyses.
Means within a column followed by different letters (superscripts: a, b, c, d) are significantly different (p<0.05).
Formula ctrl: contained all the ingredients indicated in Table 1, except the yeast autolysate and chromium. SRCL ctrl: contained only SRCL.
x%: means x% of yeast autolysate.
xC: means x of degree Celsius at the extruded die.

**Table 5. Proximate Analysis Summary, Yeast autolysate** (Lynside® Nutri Snack Plus) **and Split Red Chief Lentil (SRCL)**

| Description | Moisture, % | Fat, % | Ash, % | Protein, % | CHO, % |
|---|---|---|---|---|---|
| Lynside® Nutri Snack Plus | 3.53 | 1.06 | 2.81 | 51.29 | 41.31 |
| SRCL | 10.23 | 0.79 | 2.9 | 26.63 | 60.38 |

**Table 6. Proximate Analysis, Summary**

| Increase (%) in nutritional value due to yeast autolysate fortification | | | |
|---|---|---|---|
| **Nutrients** | **Formula Ctrl** | **12%, 140C** | **% increase** |
| **Protein** | **22.1[b]** | **25[a]** | **13.12** |
| **Ash** | **1.92[b]** | **4.27[a]** | **122.40** |
| **Fat** | **0.37[b]** | **0.64[a]** | **72.97** |

(continued)

| Increase (%) in nutritional value due to yeast autolysate fortification | | | |
|---|---|---|---|
| Nutrients | Formula Ctrl | 12%, 140C | % increase |
| IVPD | 83.40[b] | 88.60[a] | 6.24 |

Values represent mean of three replicate analyses.
Means within a column followed by different letters (superscripts: a, b) are significantly different (p<0.05).
Formula ctrl: contained all the ingredients indicated in Table 1, except the yeast autolysate and chromium.
12%, 140C: means formulation containing 12% of yeast autolysate extruded at die temperature of 140 degree Celsius.

**Table 7. In vitro Protein Digestibility (IVPD) of raw and extruded lentil and lentil fortified with yeat autolysate and chromium yeasts**

| Description | Raw product IVPD, % | Extruded product[2] IVPD, % |
|---|---|---|
| Casein (reference) | 91.081 | 91.081 |
| #1: 100% Lentil | 80.080[c*,b**] | 82.313[b,a] |
| #2: Lentil formulation without yeast autolysate | 79.840[c,b] | 87.743[a,a] |
| #3: Lentil formulation with 12% yeast autolysate | 84.367[a,b] | 88.647[a,a] |
| #4: Lentil formulation with 12% yeast autolysate + Cr[1] (1.25 ppm) | 81.950[b,b] | 87.260[a,a] |
| #5: Lentil formulation with 12% yeast autolysate + Cr[1] (5.36 ppm) | 81.770[b,b] | 88.770[a,a] |

[1]Cr: chromium provided by the product Lynside® Forte Cr2000 that comprises deactivated yeast and chromium.
Values represent mean of three replicate analyses.
Significant differences within and between columns are separated by a comma.
*Means within a column followed by different letters (superscripts: a, b, c) are significantly different (p<0.05).
**Means between columns followed by different letters are significantly different (p<0.05).
[2]extruded product: at die temperature of 140 degree Celsius.

**Table 8. Sensory Evaluation of extruded lentil and lentil fortified with yeat autolysate**

| Form | N | Mean |
|---|---|---|
| 0%, 140C | 20 | 3.700[b] |
| 4%, 140C | 40 | 5.125[a] |
| 8%, 140C | 40 | 5.050[a] |
| 12%, 140C | 40 | 5.725[a] |
| 16%, 140C | 20 | 4.900[a,b] |
| 0%, 160C | 20 | 3.250[b] |
| 4%, 160C | 40 | 5.050[a] |
| 8%, 160C | 20 | 4.600[a,b] |
| 12%, 160C | 40 | 5.375[a] |
| 16%, 160C | 40 | 5.450[a] |

Means within a column followed by different letters (superscripts: a, b) are significantly different (p<0.05).
N: number of tasters.
X%,140C; X%,160C; means formulations containing 0 - 16 % of yeast autolysate extruded at die temperatures of 140 or 160 degree Celsius.

**Table 9. Palatability, glycemic index, glycemic load, GI and GL category determined** on **extruded lentil and lentil fortified with yeat autolysate and chromium yeasts**

| Test Meal | Palatability (mm) | Glycemic Index | Glycemic Index Category[4] | Glycemic Load[5] | Glycemic Load Category[6] |
|---|---|---|---|---|---|
| Glucose Control | 57.0±9 | 100 | High | 10.0 | Low |
| [1]Lentil-1 | 50.8±8.5 | 66.9±5.3 | Medium | 12.1 | Medium |
| [2]Lentil-2 | 43.4±10.7 | 61.8±5.2 | Medium | 10.7 | Medium |
| [3]Lentil-3 | 42.9±10.5 | 62.0±4.5 | Medium | 10.6 | Medium |

[1]Snacks made from lentil-based formulations indicated in Table 1, 1st formula.
[2]Snacks made from lentil-based formulations indicated in Table 1, 2nd formula, containing 1.25 ppm chromium provided by the product Lynside® Forte Cr2000 that comprises deactivated yeast and chromium.
[3]Snacks made from lentil-based formulations indicated in Table 1, 2nd formula, containing 5.36 ppm chromium provided by the product Lynside® Forte Cr2000 that comprises deactivated yeast and chromium.
[4]Category from GI Factor (Brand-Miller et al).
[5]usual serving size=30g except for glucose where the usual serving size = 10g.
[6]GL Category calculated as GL=GI x available CHO per serving/100.

**Table 10. Values of diameter, percent variability and expansion ratio of garbanzo extrudates**

| Type of extrudate | [1]Control | | | [2]Rods | | | [3]Balls | | |
|---|---|---|---|---|---|---|---|---|---|
| Sample # | [4]Diameter (mm) | [5]Var (%) | [6]ER | [4]Diameter (mm) | [5]Var (%) | [6]ER | [4]Diameter (mm) | [5]Var (%) | [6]ER |
| 1 | 12.286 | 11.164 | 12.322 | 12.054 | 6.991 | 11.863 | 11.930 | 6.519 | 11.620 |
| 2 | 12.578 | 9.053 | 12.915 | 12.140 | 6.327 | 12.034 | 11.595 | 9.144 | 10.977 |
| 3 | 12.626 | 8.706 | 13.014 | 12.438 | 4.028 | 12.632 | 12.130 | 4.952 | 12.011 |
| 4 | 12.884 | 6.840 | 13.551 | 12.156 | 6.204 | 12.066 | 10.665 | 16.432 | 9.289 |
| 5 | 12.508 | 9.559 | 12.771 | 12.384 | 4.444 | 12.522 | 11.570 | 9.340 | 10.928 |
| 6 | 12.760 | 7.737 | 13.291 | 12.290 | 5.170 | 12.341 | 11.720 | 8.165 | 11.213 |
| 7 | 12.760 | 7.737 | 13.291 | 12.408 | 4.259 | 12.572 | 12.095 | 5.226 | 11.943 |
| 8 | 13.108 | 5.221 | 14.026 | 12.308 | 5.031 | 12.373 | 10.795 | 15.413 | 9.515 |
| 9 | 12.836 | 7.187 | 13.450 | 12.354 | 4.676 | 12.471 | 11.795 | 7.577 | 11.374 |
| 10 | 12.498 | 9.631 | 12.751 | 12.360 | 4.630 | 12.479 | 12.000 | 5.971 | 11.755 |
| 11 | 12.822 | 7.289 | 13.421 | 12.336 | 4.815 | 12.426 | 11.420 | 10.516 | 10.647 |
| 12 | 12.920 | 6.580 | 13.627 | 12.728 | 1.790 | 13.232 | 12.010 | 5.892 | 11.780 |
| 13 | 12.954 | 6.334 | 13.698 | 12.416 | 4.198 | 12.595 | 12.050 | 5.579 | 11.853 |
| 14 | 12.722 | 8.012 | 13.212 | 12.594 | 2.824 | 12.952 | 10.965 | 14.081 | 9.821 |
| 15 | 12.782 | 7.578 | 13.337 | 12.220 | 5.710 | 12.195 | 11.440 | 10.359 | 10.684 |
| 16 | 12.956 | 6.320 | 13.703 | 12.284 | 5.216 | 12.326 | 10.725 | 15.961 | 9.393 |
| 17 | 13.112 | 5.192 | 14.035 | 12.432 | 4.074 | 12.624 | 11.565 | 9.379 | 10.931 |
| 18 | 12.560 | 9.183 | 12.878 | 12.224 | 5.679 | 12.201 | 11.010 | 13.728 | 9.899 |
| 19 | 12.844 | 7.129 | 13.467 | 12.374 | 4.522 | 12.509 | 11.640 | 8.792 | 11.062 |
| 20 | 12.612 | 8.807 | 12.985 | 12.624 | 2.593 | 13.019 | 11.565 | 9.379 | 10.946 |
| 21 | 12.688 | 8.257 | 13.142 | 12.280 | 5.247 | 12.315 | 10.705 | 16.118 | 9.368 |
| 22 | 13.166 | 4.801 | 14.150 | 12.250 | 5.478 | 12.257 | 10.995 | 13.846 | 9.891 |
| 23 | 12.828 | 7.245 | 13.433 | 12.762 | 1.528 | 13.297 | 10.945 | 14.238 | 9.779 |
| 24 | 13.204 | 4.526 | 14.232 | 12.436 | 4.043 | 12.632 | 10.500 | 17.724 | 9.001 |

(continued)

| Type of extrudate | ¹Control | | | ²Rods | | | ³Balls | | |
|---|---|---|---|---|---|---|---|---|---|
| Sample # | ⁴Diameter (mm) | ⁵Var (%) | ⁶ER | ⁴Diameter (mm) | ⁵Var (%) | ⁶ER | ⁴Diameter (mm) | ⁵Var (%) | ⁶ER |
| 25 | 12.644 | 8.576 | 13.051 | 12.240 | 5.556 | 12.237 | 10.595 | 16.980 | 9.164 |
| Overall Average | **12.786** | **7.546** | **13.35** | **12.364** | **4.601** | **12.49** | **11.377** | **10.853** | **10.59** |

¹Control: Extrudate from 100 garbanzo flour
²Rods: Extrudate from garbanzo based formulation in the form of rods
³Balls: Extrudate from garbanzo based formulation in the form of balls
⁴Diameter (mm): Each diameter value in the table represent the average of five randomly measures on rod and ball extrudates
⁵Var (%): Percent variability= 100 - [(diameter value/maximum diameter value of 125 values) *100]
⁶ER: Expansion Ratio of the extrudate

**Moisture content of the feed, die temperature and pressure effect on extrudate expansion**

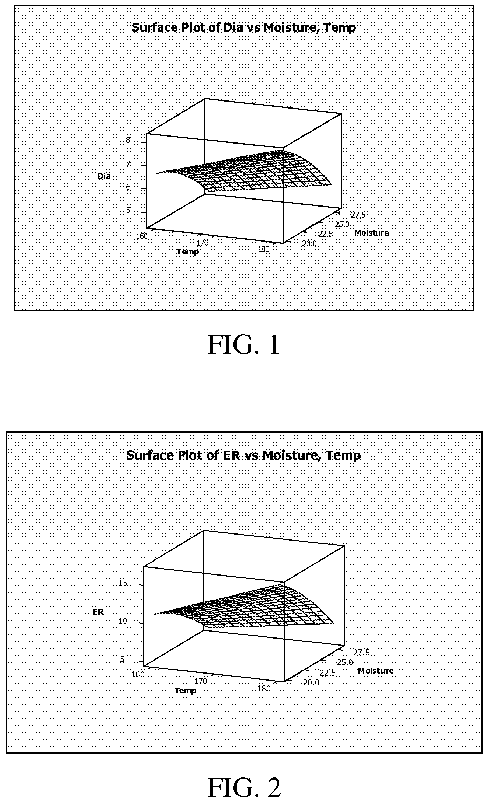

A certain amount of moisture is necessary in order to permit proper cooking and promote expansion of the extrudate (Chen et al. 1991, Gujska and Khan, 1990, Balandran et al, 1998). We determined the effect of moisture and die temperature on expansion characteristics, such as diameter and expansion ration, of lentil, dry peas and garbanzo bean extrudates. As observed in Figures 1 and 2, the diameter as well as the expansion ratio of the lentil extrudate is directly proportional to die temperature and inversely proportional to feed moisture. A similar expansion pattern was observed for dry peas and garbanzo extrudates. Additionally, the surface response graphs indicates that when the feed moisture decreased from 28 to 20%, the extrudate expanded significantly ($p \leq 0.05$) giving values of about 8 and 16 for diameter and the expansion ration, respectively. Expansion ratios of 0.91-1.89 have been reported for extruded cowpea meal (Phillips et al., 1984), 3.8 for rice/chickpea mixture (Bhattacharya and Prakash, 1994), 1.34-5.78 for extruded small white beans (Edwards et al., 1994), 1.45- 1.60 for defatted soy flour/sweet potato mixture (Iwe, 2000), 1.3-3.6 for maize/soybean mixture (Veronica, et al., 2006), which are significantly small to those obtained in our studies.

Proper expansion of the extrudate at low moisture content, typically 4 to 6% on dry basis, is desirable for the production of ready-to-eat snacks and breakfast cereal type products. Further drying may be necessary to bring the moisture to the above level for more moist extrudates to achieve proper texture, while maintaining the shelf stability of the final expanded extruded product.

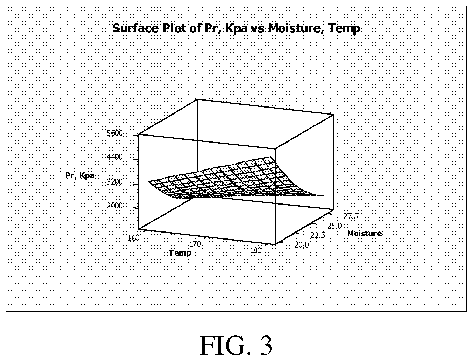

Pressure in the extruder is a function of die restriction, temperature build up along the length of the extruder barrel, and compression caused by the screw. Pressure is created when pulses-based flour is fed into the extruder and gets mixed with water and other additives to become plasticized dough, which is progressively cooked, while moving at high speed along the externally heated barrel sections of the extruder. The steam formation caused by the combined effect of moisture and temperature have a direct effect on die pressure. An important role of pressure on the product under extrusion is its direct effect on mass viscosity of the melt. The surface response plot shown in Figure 3 demonstrates that pressure, as diameter and expansion ratio of the lentil extrudate, is directly proportional to die temperature and inversely proportional to feed moisture. The observed values of 3,200-4,400 kPa falls in the range of die pressure values reported for extruded small white beans of 2,620 to 7,860 kPa (Edward et al., 1994). However, despite the largest values on die pressure in the latest study, their reported expansion ratios of 1.34-5.78 were significantly lower than 5-16, obtained in our study. This indicates that specific processing conditions of moisture and temperature among others are critical to optimize the expansion of legume based extrudates. Additionally, pressure builds up and pressure stability is indicative of proper extruder operation. Therefore, an operator may rely on pressure indicators in order to determine and monitor the effective operation of the extruder.

**Extrusion processing parameters effect on the proximate composition of legume extrudates**

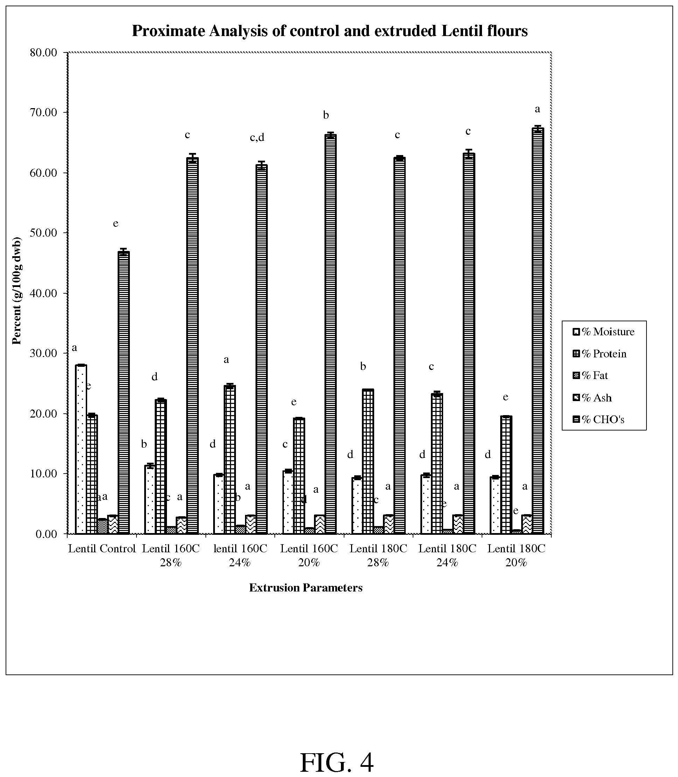

The effect of extrusion processing parameters of die temperature of 160 and 180°C and moisture addition of 28, 24, and 20% on the proximate composition of lentil flours is presented in Figure 4. The largest reduction on moisture content was observed at the highest moisture addition under both extrusion die temperatures studied. Lentil flour extruded with moisture addition in the range of 28 to 20% demonstrated a significant ($P \leq 0.05$) reduction of 55.51 and 59.69% in moisture content at the die temperatures of 160 and 180°C compared to the control flour, respectively. That is, the extrudate moisture content decreased with an increased in die temperature as well as with a reduction in feed moisture. Higher melt temperature correspond to higher vapor pressure due to the moisture present in the melt. When the melt comes out of the die the difference between the vapor pressure of the melt and atmospheric pressure is higher and thus it expands associated with flushing of water vapor, resulting in lower moisture content of the extrudate upon cooling. This phenomenon is useful because it may avoid the post-extrusion drying of the extrudate. As with feed moisture, the crude fat (extracted with petroleum ether) showed to be significantly lower ($P \leq 0.05$) on the extruded lentil flours than in the control flours.

Moisture content also has an impact on the concentration of nutritional components in the extrudate, such protein and ash. Lentils extruded with moisture addition in the range of 28 to 20%, demonstrated crude protein values of 11.46 and 12.71% at extruder die temperatures of 160 and 180°C, respectively. In general, the higher values in crude protein content were indirectly proportional to die temperatures and directly proportional to the feed moisture. Total ash (minerals) values showed only a minor increase with a reduction in moisture content in the extrudate and an increase in die temperature of the process. A similar pattern on proximate composition values was observed for dry peas and garbanzo extrudates. This indicated that the extrusion processing parameters of moisture and temperature studied, had a direct effect on the nutrient compositional values of the final extrudate. Total carbohydrate values, which were calculated by difference, varied according to the variation on proximate composition values of the analyzed nutrients from 46.83 to 67.33%.

**Moisture Content and Water Activity**

Moisture content of the melt is critical since it relates both to how much the extrudate will expand when it exits the extruder, as well as to the shelf life of the finished product. Moreover, moisture content of the extrusion product is important because it has an effect on both the shelf life of the product as well as consumer acceptance.

Water activity ($a_w$) predicts stability of foods and food ingredients with respect to physical properties, microbial growth and rates of deteriorative reactions. The latest, play a significant role in determining the activity of enzymes and vitamins in foods and can have a major impact their color, taste, and aroma. Therefore, control of $a_w$, rather than water content, is very important in the food industry as low $a_w$ presents stability of food materials under storage (increasing shelf life). Additionally, $a_w$ causes large changes in textural characteristics in the food material such as crispness and crunchiness (*e.g.* the sound produced by 'crunching' breakfast cereals and expanded snacks disappearing about $a_w \geq$ 0.65). In general Processed Foods have a $a_w$ of 0.72-0.80 with a moisture content of about 15% and Dehydrated Foods have a $a_w \leq 0.4$ with a moisture content of about 5%. The absolute limit of microbial growth is about $a_w = 0.6$.

Most commercial extruded cereal-based snacks have final moisture content in the range of 4 to 6% with $a_w \leq$ 0.4. However, in our study with legume extrudates, we found that extrudates with a moisture content between 9-11% had an $a_w$ in the range of 0.30-0.44, which fell within the range of shelf stable product. The extrudates remained shelf stable and with good texture (dry and crispy) and appearance for up to 1 year.

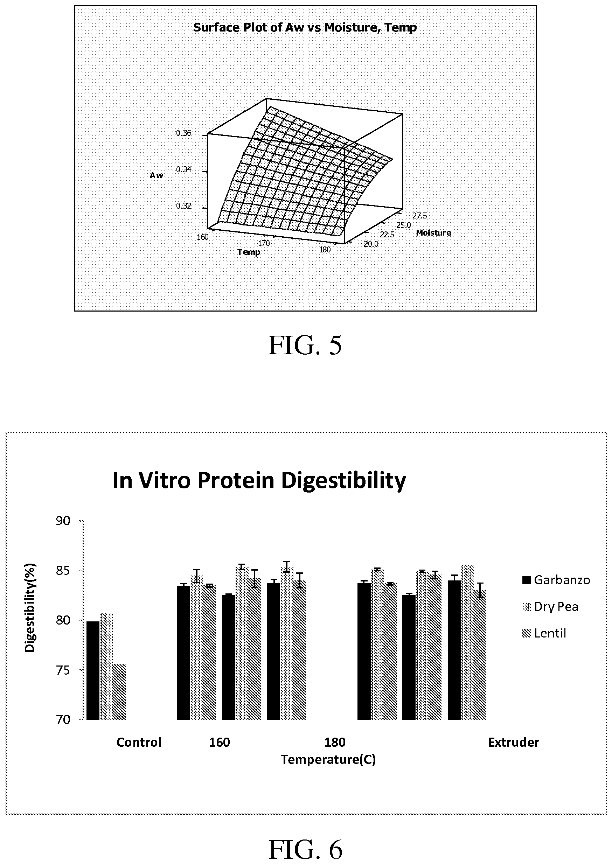

Figure 5, showed that $a_w$ varied in the range of 0.30-0.36 with variations in feed moisture content in the range of 20-28%. As the feed moisture was increased the $a_w$ value also increased sharply. At the lowest feed moisture content of 20%, the $a_w$ remained unaffected by the die temperatures under study. The effect of feed moisture was more pronounced than the die temperatures on the resulted water activity of the extrudates.

**Protein digestibility of extruded legumes**

For plant-based foods, legumes are relatively high in protein content. The exposure of proteins to high extrusion cooking temperatures may cause denaturation and other changes in the protein structure and/or to protein-protein interaction (Stanley, 1989; Phillips, 1988; Li et al. 2000). These physical changes in the protein structure results in a more digestible protein when consumed as a food. Cooking temperature, time and pressure of extrusion play important role in the protein's denaturation process.

The values of *in vitro* protein digestibility of the control (non-extruded) samples were 80.69, 79.86, and 75.63% for lentils, dry pea, and garbanzo flours, respectively. Figure 6 presents the results of *in vitro* protein digestibility of the three extruded legumes. In general, exposure of high protein legume flours to a high-temperature-short-time extrusion process demonstrated to improve the *in vitro* protein digestibility of the resulted extrudates. Additionally, the extruded parameter of moisture addition had a more significant effect ($P \leq 0.05$) than temperature on increasing the *in vitro* protein digestibility of the extruded legume flours under the conditions of this study. Dry pea extrudate demonstrated the higher values on *in vitro* protein digestibility, followed by lentil and garbanzo extrudates. Extrusion processing parameters effect on color of the extrudate

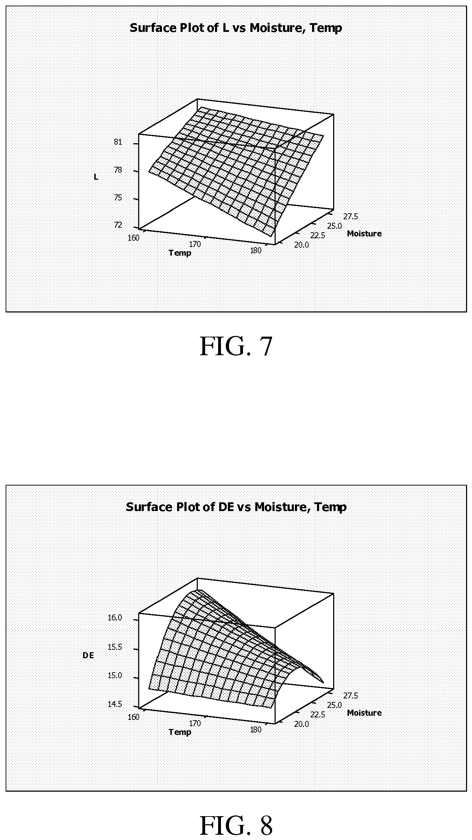

One of the effects of extrusion cooking is the change in color of the lentil extrudates. Figure 7, for example, shows that extrusion processing conditions such as moisture and temperature produce desirable color changes associated with snack type products. Lightness (L*) is a measure of color used to evaluate the acceptability of food products. Figure 7 shows that the L* of lentil extrudate was affected by die temperature and feed moisture levels, with the latter factor having more influence than the former. At higher feed moisture the L* of the extrudate was similar at all the evaluated die temperatures. Lentil extrudate exposed to lowest feed moisture of 20% and highest die temperature of 180°C, demonstrated the lowest L* values. The low processing moisture of 20% may have promoted high friction of the melt during extrusion and the high extrusion temperature of 180°C may have promoted pigment oxidation. This combined processing effect of low moisture and high temperature, is considered to be responsible for the observed discoloration in the final extrudate.

The Color index (ΔE) is an evaluation of the total color difference between the sample and control or standard by taking into consideration the color parameters L* a b*. ΔE indicates the size of the color difference but not in what way the colors are different. The response surface graph (Fig. 8) shows that ΔE increased with an increase in temperature up to about feed moisture of 24-25% and then it decreased. Overall, the effect of die temperature was more predominant on ΔE than the feed moisture range under study.

**Specific mechanical energy (SME)**

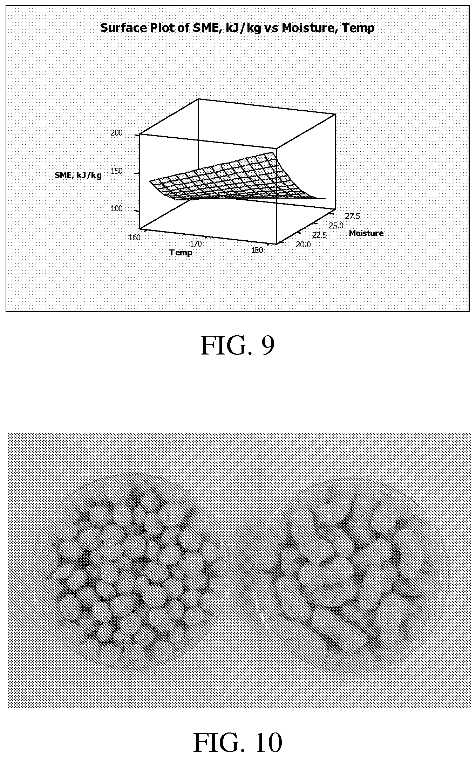

Specific mechanical energy (SME) reflects the amount of energy generated in the process of extruded pulses. The surface plot of SME as effect of moisture content of the feed and die temperature showed that the specific mechanical energy increased as the feed moisture was reduced from 28 to 20% (Fig. 9), possible at consequence of the high friction and shearing experienced by the legume based material under extrusion. Additionally, the increase in SME was more pronounced at higher temperature. Conversely, lower energy input was observed at higher feed moisture and lower temperature.

**Particle size and extruder screw speed**

To evaluate the effect of particle size and extruder screw speed on the expansion of legumes, black beans were ground using a Hammer Mill equipped with 0.85, 1.15, 1.53, and 2.28 mm stainless steel sieves and a Pin Mill to produce bean flours with different particle sizes. Pin Mill produced the finest flour. The extruder screw speeds used to process the flours were 400, 450 and 500 rpm, and the die temperature was 160°C. The flours were metered into the extruder feed port at a rate of 25 kg $h^{-1}$ and water was supplied to the extruder using a variable piston pump (Model P5-120, Bran and Luebbe, Wheeling, IL) to give a final feed moisture content of 18% (wwb).

Table 1 summarizes the average values with their corresponding standard deviations of percent torque and expansion ratio of the bean flours extruded under the different particle sizes and screw speeds studied. Percent torque and expansion ratio, within the different particle sizes evaluated, increased with an increase in screw speed. Greater expansion of extruded material is related to crispiness and therefore it is considered as a desirable attribute in the fabrication of snacks and ready to eat (RTE) foods. The fine Pin milled flours extruded at 500 rpm demonstrated the greater expansion in this study, which represented an expansion ratio of 6.74 $\pm$ 0.86.

**Table 11. Average Values of Percent Torque and Expansion Ratio, of Black Bean Flours Extruded Under Different Particle Sizes and Screw Speeds**

| | Screw Speed | Pin-milled | 0.85mm | 1.15mm | 1.53mm | 2.28mm |
|---|---|---|---|---|---|---|
| **Torque (%)** | 400rpm | 66.10±0.74 | 72.40±1.07 | 72.70±0.67 | 69.50±1.58 | 67.60±1.07 |
| | 450rpm | 67.20±0.79 | 71.50±1.08 | 72.60±1.17 | 70.20±1.03 | 65.80±0.92 |
| | 500rpm | 72.20±0.79 | 77.50±1.72 | 76.00±1.25 | 72.50±1.35 | 69.00±1.25 |
| **Expansion Ratio** | 400rpm | 6.29±0.66 | 5.58±0.75 | 4.99±0.52 | 4.76±0.47 | 4.75±0.57 |
| | 450rpm | 6.33±0.47 | 5.81±0.81 | 5.08±0.59 | 4.90±0.30 | 4.71±0.53 |
| | 500rpm | 6.74±0.86 | 6.17±0.62 | 5.52±0.71 | 5.12±0.49 | 5.08±0.46 |

**Cutting speed effect on shape and properties of legume extrudates**

Variation of cutter blade speed produced extrudates with distinct shapes. At cutter speed of about 500 rpm the extrudate was in the form of cylindrical rods were at a higher speed of about 2,000 rpm it was in the form balls or spherical shaped product (Fig. 10). Given the shapes demonstrated with the cutting speeds disclosed, one of skill in the art can manipulate the speed to obtain a variety of desired shapes. The effect of cutter speed on some physicochemical properties of the extrudate are presented in Table 2.

The taste testing of the extruded in the form of rods and balls was done to compare their sensory attributes. The results were as given in Table 3. It was observed that the sensory attributes evaluated for the two extruded products were not significantly different from each other. In spite of their different shape, the panelists gave the same score for flavor, color, texture and taste to both products indicating that they were considered equally acceptable.

**Table 12. Properties of extrudate as effect of cutter speed at fixed angle of inclination**

| Variable | Speed | Mean | SE Mean | St. Dev | CV | Min | Max |
|---|---|---|---|---|---|---|---|
| Tap Density** | Low | 64.193 | 0.926 | 2.929 | 4.51 | 61.17 | 69.68 |
| | High | 74.21 | 0.497 | 1.57 | 2.12 | 72.62 | 77.25 |
| Glass bead density[ns] | Low | 115.33 | 2.7 | 8.55 | 7.41 | 102.45 | 130.73 |
| | High | 120.73 | 4.59 | 14.52 | 12.02 | 108.78 | 159.85 |
| WAI[ns] | Low | 256.81 | 5.58 | 9.66 | 3.76 | 246.68 | 265.93 |

(continued)

| Variable | Speed | Mean | SE Mean | St. Dev | CV | Min | Max |
|---|---|---|---|---|---|---|---|
| | High | 237.41 | 7.26 | 12.58 | 5.3 | 227.39 | 251.53 |
| WSI[ns] | Low | 2.6603 | 0.0653 | 0.1131 | 4.25 | 2.55 | 2.776 |
| | High | 2.823 | 0.137 | 0.237 | 8.39 | 2.651 | 3.093 |
| WHC[ns] | Low | 545.04 | 8.01 | 11.33 | 2.08 | 537.02 | 553.05 |
| | High | 563.1 | 13.4 | 18.9 | 3.36 | 549.7 | 576.5 |
| WA[ns] | Low | 0.4218 | 0.0041 | 0.00918 | 2.18 | 0.41 | 0.433 |
| | High | 0.4282 | 0.00372 | 0.00832 | 1.94 | 0.417 | 0.439 |
| Mean D** | Low | 11.064 | 0.0655 | 0.463 | 4.18 | 10.21 | 12.04 |
| | High | 9.986 | 0.108 | 0.766 | 7.67 | 8.73 | 12.21 |
| SEI[ns] | Low | 10.484 | 0.124 | 0.875 | 8.34 | 8.92 | 12.39 |
| | High | 10.701 | 0.207 | 1.466 | 13.7 | 7.68 | 14.36 |
| Hardness**g | Low | 2494 | 112 | 709 | 28.42 | 1255 | 4433 |
| | High | 1668 | 70.6 | 440.9 | 26.43 | 662.2 | 2507 |
| Fracturability** | Low | 2577 | 138 | 875 | 33.94 | 1156 | 5112 |
| | High | 1643.6 | 58.9 | 367.6 | 22.37 | 690.6 | 2529.9 |
| Springiness** | Low | 0.23363 | 0.00436 | 0.025758 | 11.8 | 0.178 | 0.321 |
| | High | 1.523 | 0.33 | 2.061 | 135.3 | 0.21 | 5.6 |
| Cohesiveness** | Low | 0.06603 | 0.00353 | 0.02236 | 33.86 | 0.02 | 0.134 |
| | High | 0.09974 | 0.00491 | 0.03065 | 30.73 | 0.05 | 0.16 |
| Guminess[ns] | Low | 174.9 | 17.7 | 112.2 | 64.14 | 39.4 | 593.7 |
| | High | 176.2 | 14.5 | 90.7 | 51.48 | 30.8 | 374.1 |
| Chewiness** | Low | 41.97 | 4.75 | 30.06 | 71.62 | 9.52 | 163.9 |
| | High | 240.4 | 60.1 | 375.1 | 156.01 | 14.7 | 1313.6 |
| Resilience** | Low | 0.04575 | 0.00213 | 0.01348 | 29.46 | 0.017 | 0.081 |
| | High | 0.07872 | 0.0036 | 0.0225 | 28.58 | 0.04 | 0.12 |
| Sphericity[1] | High | 0.95 | | 0.03 | 2.69 | | |

** $P > 0.01$,
ns= not significant.
1= only for ball shaped product.

**Table 13. Sensory attributes of extrudates as effect of cutter speed at fixed angle of inclination**

| Property | Cutter speed | Mean | SD | SE Mean |
|---|---|---|---|---|
| Appearance[ns] | Low (Rods) | 6.25 | 1.183 | 0.296 |
| | High (Balls) | 5.813 | 1.109 | 0.277 |
| Color [ns] | Low (Rods) | 6.375 | 1.455 | 0.364 |
| | High (Balls) | 6.00 | 1.366 | 0.342 |
| Flavor [ns] | Low (Rods) | 6.625 | 1.31 | 0.328 |
| | High (Balls) | 6.313 | 1.25 | 0.313 |

(continued)

| Property | Cutter speed | Mean | SD | SE Mean |
|---|---|---|---|---|
| Texture [ns] | Low (Rods) | 6.75 | 1.238 | 0.31 |
| | High (Balls) | 6.063 | 0.929 | 0.232 |
| Taste [ns] | Low (Rods) | 6.563 | 1.711 | 0.428 |
| | High (Balls) | 5.875 | 1.668 | 0.417 |

## EXAMPLES

### Example 1

### Effect of Screw speed and starch sources

Decorticated Red Chief lentils (Lens culinaris L.) were obtained from Moscow Idaho Seed Co., Moscow, ID. Prior to milling, each lot of seeds was mixed to a uniform lot. For the production of flours, the homogenized lentils were ground in a hammer mill using a 1 mm screen. The lentil flower was mixed with apple fiber, high amylose corn starch and flavoring ingredients (Table 4).

A Clextral Evolum HT 32H twin-screw extrusion system (Clextral-Bivis, Firminy Cedex, France) was used in this study. The heating profiles for the six barrel sections of the extruder were 15, 80, 100, 120, 140, and 160°C, respectively. Flours were fed into the extruder feed port by a twin-screw, lost-in-weight gravimetric feeder (Model LWFD5-20, K-Tron Corporation, Pitman, NJ) at a rate of 25 kg/h and the extruder was run at three screw speeds of 500, 600 and 700 rpm. Water was added into the extruder through a variable piston pump (Model P5-120, Bran and Luebbe, Wheeling, IL) to bring the moisture contend of the feed under extrusion to 15% (wwb). When the processing conditions of torque and temperature were at steady state the extrudates, coming out of 2 circular dies 3 mm in diameter, were collected for 5 min.

**Table 14. Composition of lentil flours formulated with different starches (%, w/w)**

| Sample for lentil (%) | Lentil | Hylon V | PP40 | PC10 | PB800 | Apple Fiber | Salt | Sugar |
|---|---|---|---|---|---|---|---|---|
| 60%-Hylon V | 60 | 20 | 0 | 0 | 0 | 10 | 5 | 5 |
| 60%-PP40 | 60 | 0 | 20 | 0 | 0 | 10 | 5 | 5 |
| 60%-PC10 | 60 | 0 | 0 | 20 | 0 | 10 | 5 | 5 |
| 60%-PB800 | 60 | 0 | 0 | 0 | 20 | 10 | 5 | 5 |
| 80% Control | 80 | 0 | 0 | 0 | 0 | 10 | 5 | 5 |
| 100% Control | 100 | 0 | 0 | 0 | 0 | 0 | 0 | 0 |

The extrudates in the form of rods or flours were used to evaluate the effect of screw speed and starch sources on various physical characteristics of the product.

(EI). A digital caliper with an accuracy of $\pm 0.01$ mm was used to measure the cross sectional diameter (mm) of extrudates when the extrudates reached ambient temperature. The average value of twenty measurements for the random profiles of the same section was recorded. Expansion index was calculated as expressed as the ratio between the cross-sectional area of the extrudate and the area of the die orifice.

Product density (D). The mass of ten pieces of extrudates was measured with an accuracy of $\pm$ 0.0001g. The lengths and mean diameters of the samples were measured with the digital caliper. The density of extrudate that was assumed to be cylindrical shape in this study was calculated by the following equation:

$$D = \frac{4000000 \times M}{\pi \times h \times d^2}$$

where D is the density of extrudates (kg/m3); M is the mass of the extrudate (g); and h is the length of the extrudate (mm); d is the mean diameter from three measurements of the extrudate (mm).

Water solubility index (WSI) and water absorption index (WAI) were determined with the use of the method described by Jin et al. (1995) with minor modifications. The extrudates were ground through an Udy cyclone mill (Fort Collins, CO) with a 0.5 mm screen. A two-gram sample was dispersed into 20-mL distilled water at 25°C. The suspension in a weighted centrifuge tube was stirred vigorously on a vortex mixer for 5 sec. The tube was then kept still for 10 min and stirred for 5 sec every 5 min. The suspension was centrifuged at 3000xg for 10 min and then decanted to determine solid content in the supernatant and weigh the sediment. WSI (%) and WAI (%) were calculated as follows:

$$\text{WSI}\,(\%) = 100 \times (\text{Weight of dissolved solids in supernant})/(\text{Weight of dry solids})$$

$$\text{WAI}\,(\%) = 100 \times (\text{Weight of sediment})/(\text{Weight of dry solids})$$

Rapid viscosity analysis (RVA). The results of RVA demonstrate the changes in viscosity over a time-temperature profile, which reflects the molecular weight and conformation of starches. RVA for Red Chief lentil flours and four starches was conducted through a Rapid Visco-Analyser (RVA3d, Newport Scientific, Sydney, Australia) after a sample of 3.00g (d.b) dissolved into 25.00 g distilled water completely. All samples were subjected to a time-temperature profile described as follows. The samples were first kept equilibration at 50°C for 2 min, and then were ramped to 95°C within 9 min and held at 95°C for 15 min. The samples were in turn cooled down to 50°C within 9 min and held at 50°C for 10 min. The viscosity of samples was expressed as rapid viscosity units (RVU).

The parameters that were useful to describe to change of viscosity were recorded during measurement. Peak viscosity and peak time indicated the maximum viscosity during pasting and the time when the peak viscosity appears, respectively. Holding strength and breakdown viscosity showed the holding viscosity after the peak viscosity and the difference between the peak viscosity and the minimum viscosity during pasting, respectively. Setback demonstrated the difference between the maximum viscosity during cooling and the minimum viscosity during pasting; and final viscosity indicated the viscosity of the suspensions at the end of the RVA run (45 min). All measurements were performed in triplicate.

Texture analysis. A TA-XT2 texture analyzer (Stable Micro Systems, Surrey, England) was used to measure the texture of a cylindrical extrudate sample with a length of 10 mm at ambient temperature. A cylinder aluminum probe with a diameter of 50 mm was used to press the sample against a flat plate fixed on the loading frame to 50% of its original length at a speed of 0.5mm/s. The corresponding force-time curve was recorded and analyzed by a computer program (Texture Expert Exceed, Stable Micro Systems, Surrey, England) simultaneously. The force was recorded in gram and converted to Newton for the calculation of hardness and strength. The hardness of samples was defined as the peak value of the compression force. The sample strength was calculated by the following equation:

$$S = \frac{A_c}{t \times A_p}$$

where S is the strength (N.mm$^{-2}$), $A_c$ is the area under time-force curve (N.t), $Ap$ is the original across-sectional area of the extrudates (mm$^{-2}$) and $t$ is the time that the probe compresses on the extrudate.
Ten replications were performed to complete this calculation.

Statistical analysis. All the values of averages, standard deviations and correlations were calculated using Microsoft Excel software (Version 2002). Correlation between the physical parameters studied, were from pool values of extrudates with and without starch addition. The determination of ANOVA was performed using SAS 8.1 software (SAS, 1999) with a significant level of 5%.

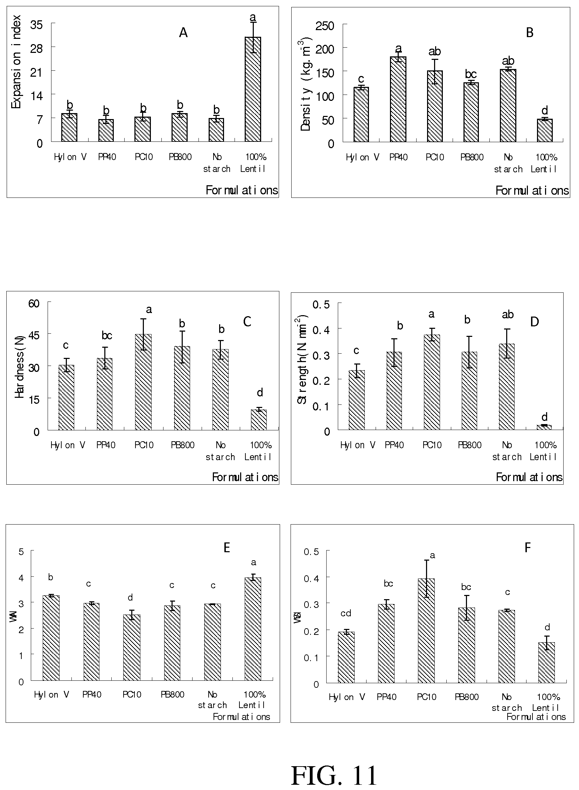

Effect of starch and fiber on the physicochemical properties of extrudates: The expansion, texture and hydration properties of the control lentil extrudate and those lentil extrudates with apple fiber and flavoring ingredients and with or without starch sources, processed at extruder screw speed of 600 rpm are shown in Figure 11 (A-F). Based on a previous study (not reported) we dermined that the effect of the flavoring ingredients salt and sugar, at the concentration used in this study, did not have a significant effect on the physicochemical properties of legume extrudates. Their inclusion in the lentil formulation was considered as standard practice in the fabrication of commercial snack type products. Therefore, the discussion below will not consider the effect of these ingredients on the physicochemical properties of the lentil extrudates studied.

Expansion: Figure 11A indicated that fiber addition significantly affected EI in this study (P < 0.05). The EI of the lentil extrudates without the addition of apple fiber was 30.7; the EI of lentil extrudates with apple fiber addition was only 6.6; while the EI of lentil extrudate formulated with the various starch sources were in the range of 6.6 to 8.2. This demonstrated that the fiber addition had a greater significant (P < 0.05) effect on EI of the lentil extrudate than the all of the starch sources evaluated. The detrimental effect of fiber on EI of the lentil extrudate could be attributed to the fact that fiber decreased the starch content in the dough.

EI of the lentil extrudate with high amylose corn starch (Hylon V) addition was slightly higher than the lentil exudates with potato starch source. It has been reported that the EI of potato flour was lower than that of corn flour, processed at the same extrusion conditions (Onwulata et al., 2001b). This could be explained as follows: (1) the gelatinization temperature of potato starch (56-66°C) is known to be lower than that of corn starch (62-72°C); the relatively low gelatinization temperature means that potato starch exhibits high melting viscosity and early melt during extrusion (Della Valle et al. 1995; Sigh et al, 2002); (2) potato starch has more phosphate cross-linkages in the amylopectin also attribute to the relatively high initial viscosity (Eerlingen et al., 1997) and low expansion during extrusion.

Density: The density of the lentil extrudate without apple fiber addition was significantly (P < 0.05) smaller than the lentil extrudates with apple fiber. Among the lentil extrudates with apple fiber and starch addition, the one with high amylose corn starch (Hylon V) had the lowest density followed by the one with modified potato starch (PB800). The highest density was observed for lentil extrudates with PP40, PC10 and lentil extrudate without starch addition (Fig. 11B).

Hardness and strength: As shown in Figures 1C and ID, the hardness and strength for the extruded lentil control samples were significantly lower (P < 0.05) than that of lentil extrudates with apple fiber, but without starch addition. Also, the extruded lentil controls were significantly lower (P < 0.05) that the lentil extrudates with apple fiber and starch addition. The lowest and highest values in hardness and strength among the lentil extrudates with apple fiber and starch addition were those with Hylon V and PC10, respectively. Additionally, no significant difference (P < 0.05) in either hardness or strength was observed for lentil extrudates with PP40 and PB800 starch addition or the lentil extrudate without starch addition. This demonstrates that the source and type of starch have significant effect on the hardness and strength of the final extrudate. It also indicated that extrudates with potato starch addition exhibited stronger (tougher) texture compared to those extrudates with high amylose corn starch (Hylon V).

Hydration properties of extrudates: Figure 11E showed that the WAI and WSI for the extruded lentil control samples were significantly different (P < 0.05) and inversely related. The WAI and WSI for lentil extrudates with apple fiber, but without starch addition, were similar. However, the WAI for the lentil extrudates, with apple fiber and starch addition, varied significantly among them and it was inversely related to the values of WAI of those extrudates. Extruded lentil control and that with Hylon V starch addition showed the highest values of WAI, while the extrudate with PC10 starch addition showed the highest value of WSI.

Properties of starch and lentil flours: Table 7 shows the RVA and the hydration properties for the lentil extrudates formulated with corn and potato starches and the control extruded lentil flour. As indicated in Table 2, the extruded lentil flours formulated with PP40 (pregelatinized potato starch) and PC10 (native potato starch) exhibited significantly (P < 0.05) the highest values of peak viscosity, holding strength, breakdown and final viscosity and setback than those formulated with others starch sources and the control. Additionally, extruded lentil flours formulated with Hylon V (high amylose corn starch) exhibited significantly (P < 0.05) the lowest values of the RVA parameters of the studied starches.

**Table 15. Effect of starch sources on RVA parameters, WAI and WSI of lentil based extrudates**

|  | Peak Viscosity | Holding strength | Breakdown | Final Viscosity | Setback | Peak time | WAI | WSI |
|---|---|---|---|---|---|---|---|---|
| Hylon V | 33.89c | 34.06c | -0.17b | 49.36b | 15.31b | 12.93a | 2.37b | 0.01b |
| PP40 | 871.92a | 248.17b | 396.78a | 418.89a | 173.72a | 5.29b | 9.80a | 0.00b |
| PC10 | 827.61b | 307.64a | 520.17a | 445.39a | 137.75a | 6.74b | 2.11b | 0.01b |
| PB800 | 95.42c | 42.36c | 53.06b | 66.69b | 24.34b | 6.60b | 2.05b | 0.00b |
| Lentil | 27.00c | 2.06d | 24.95b | 118.00b | 115.95a | 7.00b | 1.99b | 0.38a |

*Different letters (a, b and c) indicated significant (P <0.05) differences.

Table 7, also shows that the different starch sources had great influence on the WAI and WSI of the lentil based extrudates. The highest value of WAI was observed for the extruded lentil flours formulated with PP40 starch and the lowest for the lentil flours. With respect to WSI, the highest (P <0.05) value was observed for the extruded lentil flour. The extruded lentil flours formulated with the various starches were not significantly different (P < 0.05) among themselves.

The correlation between the RVA and hydration properties with other physical parameters of lentil extrudates studied is shown in Table 16. Among the RVA parameters, setback had a significant negative correlation with expansion and a positive correlation with density of the extrudates. The correlation between the stated physical properties of the extrudates among all other samples varied randomly and was lower than the one previously observed for setback.

**Table 16. Correlation between the RVA and hydration properties with other physical parameters of lentil extrudates**

| | lentil extrudates | | | | | | | |
| --- | --- | --- | --- | --- | --- | --- | --- | --- |
| | Peak Viscosity | Holding strength | Breakdown | Final Viscosity | Setback | Peak time | WAI | WSI |
| Hardness | 0.79 | 0.75 | 0.81 | 0.81 | 0.73 | -0.86 | -0.68 | -0.04 |
| Strength | 0.68 | 0.62 | 0.72 | 0.77 | 0.89 | -0.87 | -0.78 | 0.29 |
| Expansion | -0.27 | -0.20 | -0.31 | -0.45 | -0.94 | 0.57 | 0.68 | -0.79 |
| Density | 0.45 | 0.37 | 0.49 | 0.60 | 0.95 | -0.72 | -0.75 | 0.65 |
| WAI | -0.19 | -0.12 | -0.23 | -0.23 | -0.42 | 0.48 | 0.36 | -0.29 |
| WSI | 0.54 | 0.48 | 0.57 | 0.58 | 0.66 | -0.38 | -0.28 | 0.18 |

Based on the result of the physicochemical evaluation of the extrudates described above, we determined the effect of different extruder screw speeds on the physicochemical properties of the lentil extrudate with Hylon V starch and apple fiber.

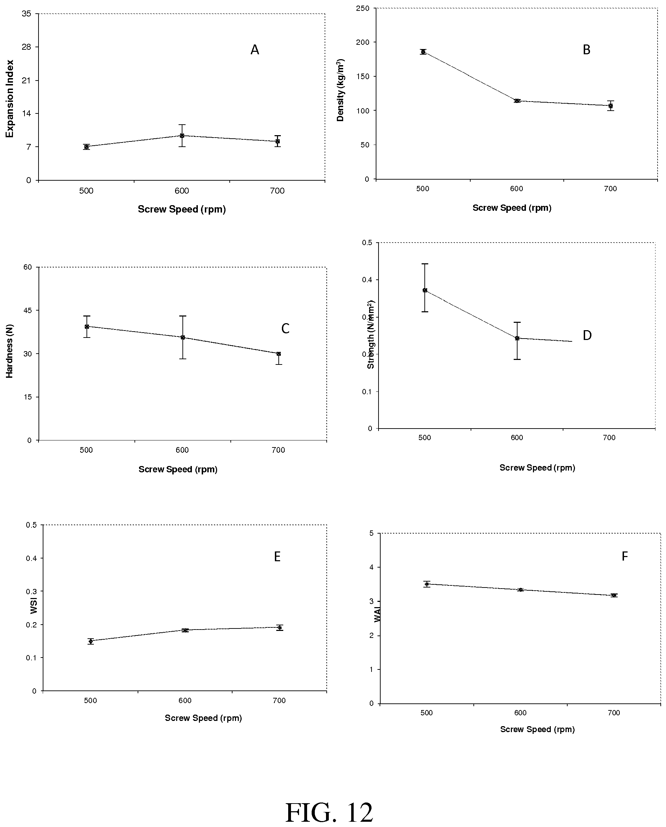

Screw speed and physicochemical properties of extrudates: The effects of screw speed on the physicochemical properties of the lentil extrudate with hylon V starch and apple fiber are shown in figure 12 (A-F). For this particular section, on we will refer the lentil extrudate with hylon V starch and apple fiber as the extrudate.

Expansion Index: As shown in figure 12A, increase in extruder screw speed from 500 rpm to 600 rpm largely raised the Expansion Index (EI) of the extrudate from 6.5 to 8.9. But, there was little change in EI when the screw speed was increased from 600 to 700 rpm. Even though the EI was highest at screw speed of 600 rpm, those values were not significantly different ($P < 0.05$) than the values of EI at 500 or 700 rpm due to the observed variability of the data at screw speed of 600 rpm. This observed data variability could have been due to less uniformity of the extrudate rod at this particular screw speed or to the inclusion of outliers in the data. In general, this information demonstrated that extruder screw speed influenced the expansion of legume based extrudates. Similarly, it has been reported that screw speed the expansion of corn meal based extrudates increased with an increase in extruder screw speed (Jin et al., 1995). Additionally, it was reported that high shear stress (due to high screw speed) increased the elasticity and decreased the viscosity of the starch dough (Della Valle et al., 1997), which could be related to improved expansion of cereal extrudates (Padmanbhan and Bhattacharya, 1989; Ilo et al., 1996). Conversely, it was reported that high shear stress brought by high screw speed induced more starch degradation and resulted in less expansion on starch extrudates (Van Den Einde et al., 2003). It our study, starch degradation on the extrudates was not evaluated. However, based on the fact that the EI of the extrudate showed to decrease when the screw speed increased from 600 to 700 rpm tend to corroborate with the increase on starch degradation observed by the previous authors on starch extrudates, at a consequence of high screw speed. Additionally, our study indicates that there is a limited in screw speed to favor expansion above which the expansion of the extrudate decreases.

Density: Figure 12B showed a drop in density of the extrudate associated with an increase in screw speed. Contrary to the observed variability in the data of expansion at 600 rpm, the data here was very uniform. This tends to indicate that the variability on expansion data at 600 rpm was due to the inclusion of outliers in the data and not to the lack of uniformity of the extuded rod. The drop in density (Fig. 12B) was inversely related to the observed increased in expansion of the extrudate (Fig. 12A). A similar negative relationship between density and expansion was also reported by Onwulata et al. (2001a) for corn extrudates. This inversed relationship between density and expansion can be use as a tool in the development of highly expanded low-density legume based extruded products.

The Hardness and strength: Figure 12C and 12D demonstrated that increase in screw speed from 500 rpm to 700 rpm induced a remarkable drop in the hardness and strength of the extrudates. The significance of the data at the different screw speed was affected by the observed variability of the data. Additionally, this variability was larger at 500 and 600 rpm than at 700 rpm. Instrument sensitivity could have induced this observed variability. This could have been improved by using more than the 10 repetitions used in this study, which indicates the need for the development of a standard methodology for this measurement.

WSI and WAI: As observed with the expansion parameter (Fig. 12A), increase in screw speed from 500 to 700 rpm was accompanied with an increase in WSI of the extrudate (Fig. 12E). Also, this increased in WSI was inversely related to the observed decreased in WAI (Fig. 12F) and density of the extrudate (Fig. 12B). This indicates that the physicochemical composition of extruded flours was affected by the screw speed of the process. Since WSI is related to the quantity of soluble molecules and starch dextrinization, the increased in WSI with increased in screw speed could be associated to a mayor degradation of the starch in the extrudate as the screw speed increased from 500 to 700 rpm.

Uncooked starch does not absorb water at room temperature. Therefore, it not swell and its viscosity is significantly lower that cooked-gelatinized starch. The relative high values of WAI are related to the water absorption by the flour extrudate and to gel formation. Additionally, the small variation in WAI values observed at the different screw speeds indicate that the extrudate was equally cooked under the screw speeds and processing condition of this study.

**Example 2**

**Leavening agent and high amylose corn starch effect**

Lentil beans (*Lens esculenta*), garbanzo beans (*Cicer arientinum* L.), whole yellow dry peas, and split-decorticated yellow dry peas (*Pisum sativum*) with moisture content of 9.2, 8.6, 9.6, and 10.1 % (wb), respectively, were individually mixed to uniform lots and ground to flour using a Pin Mill model 160Z (Alpine, Co. Augsburg, Germany). Sodium bicarbonate (Sigma Chemical Co. St. Louis, MO) and starch Hylon V (National Starch & Chemical, Bridgewater, NJ) were added to flours at 0.4% and 20% (w/w), respectively (Table 17). The flours with added ingredients were mixed for 10 min using a large Hobart mixer Model V-1401 (The Hobart Mfg. Co., Troy, OH) before extrusion processing. Totally 2,000 lbs of legume seeds and 350 lbs of starch were used in this comprehensive extrusion experiment.

**Table 17. Legume flours formulated with leavening agent and high amylase corn starch**

| Legume and ingredients | Legume (%) | NaHCO$_3$ (%) | Hylon V (%) |
|---|---|---|---|
| Lentil | 100 | 0 | 0 |
| Lentil - LA[1] | 99.6 | 0.4 | 0 |
| Lentil - St[2] | 80 | 0 | 20 |
| Lentil - (LA + St) | 79.6 | 0.4 | 20 |
| Garbanzo | 100 | 0 | 0 |
| Garbanzo - LA[1] | 99.6 | 0.4 | 0 |
| Garbanzo - St[2] | 80 | 0 | 20 |
| Garbanzo - (LA + St) | 79.6 | 0.4 | 20 |
| Whole pea | 100 | 0 | 0 |
| Whole pea - LA[1] | 99.6 | 0.4 | 0 |
| Whole pea - St[2] | 80 | 0 | 20 |
| Whole pea - (LA +St) | 79.6 | 0.4 | 20 |
| Split pea[3] | 100 | 0 | 0 |
| Split pea - LA[1] | 99.6 | 0.4 | 0 |
| Split pea - St[2] | 80 | 0 | 20 |
| Split Pea - (LA + St) | 79.6 | 0.4 | 20 |

[1]Leavening agent (LA): sodium bicarbonate.
[2]Starch (St): Hylon V, a high amylase corn starch.
[3]Split pea: Split and decorticated dry pea.

A twin-screw extruder (Continua 37, Werner and Pfleiderer Corp., Ramsey, NJ) system was used to process the legume flours. The extruder had eight barrel sections, each with a length of 160 mm. The screw diameter was 37 mm and the total configured screw length was 1,321 mm, which gave an overall L/D ratio of 35.7. Each barrel section was heated by separate hot oil recirculating systems (Model MK4X06-TI, Mokon Div., Protective Closures Co., Inc., Buffalo, NY). The heating profile used in this study was: no heat, 60, 80 100, 100, 120, 140, and 160°C corresponding to barrel sections 1 to 8, respectively. Screws were driven by an 11.2 kW variable speed DC drive (Model DC300, General Electric Co., Erie, PA) operated at 500 rpm. The entire system was controlled by a programmable controller (Series One Plus, General Electric Co., Charlottesville, VA). Flour was metered into the feed port by a twin-screw, lost-in-weight gravimetric feeder (Model LWFD5-20. K-Tron Corp., Pitman, NJ) at a rate of 25 kg h$^{-1}$ (wwb), and water was supplied to the extruder using a variable piston pump (Model P5-120, Bran and Luebbe, Wheeling, IL) to give a final moisture content of 15% (wwb) to the feed solids. Legume flours were extruded trough a die containing two circular openings 3.5 mm in diameter. A computer collected extruder parameters' data at a 1 s intervals for a total of 5 min, using LabView data acquisition system version 5.0 (National Instruments, Austin, TX). Data were collected approximately 5 min after the operation conditions of torque and pressure were at steady state.

Cross sectional diameter was measured with a digital caliper in mm at two random places on the extruded material, without cutting, in the form of rods coming out of the extruder die. A total of 20 measurements were made per each extrusion run and the expansion ratio of the legume extrudate (rods) was calculated by dividing the cross sectional area of the extrudates by the cross sectional area of the 3.5mm die orifices. After measurements, the extruded material was collected in large plastic bags placed in 20 gal plastic cans, cooled down to room temperature, and weighed before stored at refrigeration temperature for subsequent sample preparation and analyses.

Diameter and expansion of extrudates: The average data of diameter and expansion ration of the extrudates is presented in Table 18. The average diameter data was directly proportional to the average expansion ratio data. This was because the calculation of expansion ratio depended on the radio of the diameter of the extrudate. In general the expansion ratio was highest for split pea and lowest for garbanzo extrudates. In increasing order of magnitude, the expansion ratio of the legume extrudates was as followed: split pea > whole pea > lentil > garbanzo.

**Table 18. Extrudate diameter measurements and expansion ratio**

| Extruded Product | Average Diameter of Extrudate[1] (mm) | Expansion ratio |
|---|---|---|
| Lentil | 10.94±0.49 | 9.77 |
| Lentil - LA[2] | 10.51±0.41 | 9.02 |
| Lentil - St[3] | 13.95±0.54 | 15.89 |
| Lentil - (LA + St) | 13.25±0.63 | 14.33 |
| | | |
| Garbanzo | 4.57±0.12 | 1.70 |
| Garbanzo - LA[2] | 4.11±0.19 | 1.38 |
| Garbanzo - St[3] | 7.57±0.62 | 4.68 |
| Garbanzo - (LA + St) | 6.94±0.51 | 3.93 |
| | | |
| Whole pea | 12.35±0.79 | 12.45 |
| Whole pea - LA[2] | 11.92±0.70 | 11.60 |
| Whole pea - St[3] | 14.20±0.57 | 16.46 |
| Whole pea - (LA +St) | 14.69±0.66 | 17.62 |
| | | |
| Split pea[4] | 15.93±0.53 | 20.72 |
| Split pea - LA[2] | 15.77±0.96 | 20.30 |
| Split pea - St[3] | 17.22±1.22 | 24.21 |
| Split Pea - (LA + St) | 17.20±1.36 | 24.15 |

[1] Mean and standard deviation of 20 measurements
[2] Leavening agent (LA): sodium bicarbonate added at 0.4% (w/w).
[3] Starch (St): Hylon V added at 20% (w/w).
[4] Split pea: Split and decorticated dry pea.

The addition of high amylose corn starch to the legume flours increased the expansion ratio in 2.75, 1.63, 1.32, and 1.17 times for garbanzo beans, lentils, whole peas, and split pea extrudates, respectively. Conversely, the addition of sodium bicarbonate slightly reduced the expansion ratio of the extruded products.

Table 19 represent the effect of the legume extrudates on the extrusion processing parameters of die temperature, die pressure and torque. In general it was observed that the different legumes and legume formulated with leavening agent and/or high amylose corn starch had a highly uniform effect on the studied extrusion processing parameters. Also, it was observed that the torque, generated at consequence of the process, was directly related to the die pressure. The extrusion temperature profile was set to have 160°C on the last barrel section. However, with the exception of garbanzo extrudates, the values of die temperature for the legume extrudates were above 160 °C, regardless of the type of seed or ingredient in the formulation. This indicates that there was additional heat generated during the process, in the form of mechanical heat, as a consequence of shearing and pressure. The die temperature for the different garbanzo extrudates was below 160 °C, which indicated that first, there was not additional heat generated during the process of these extudates; and second, that the feed material promoted a small cooling effect on the process. Garbanzo bean contain about 5 percent fat, which was more that double the amount of fat present in the other studied legumes. Therefore, the melting of the fat during processing may have act as a lubricant on the screws promoting less shearing effect. Additionally, the lowest values of torque and die pressure observed for these extrudates further indicate that the lubrication action of the melted fat flowed easier and expanded less that all the other studied legumes.

**Table 19. Extrusion processing parameters**

| Product from | Extrusion parameters | | |
| --- | --- | --- | --- |
| | Die Temperature (°C) | Torque (%) | Die Pressure (psi) |
| Lentil | 176.55±1.02 | 66-68 | 230-300 |
| Lentil - LA[1] | 179.18±0.60 | 64-66 | 210-310 |
| Lentil - St[2] | 171.06±0.83 | 69-72 | 140-280 |
| Lentil - (LA + St) | 173.18±1.62 | 68-72 | 130-290 |
| Garbanzo | 151.24±0.29 | 48-50 | 140-220 |
| Garbanzo - LA[1] | 150.29±0.19 | 45-47 | 130-200 |
| Garbanzo - St[2] | 156.59±0.60 | 53-55 | 120-220 |
| Garbanzo - (LA + St) | 154.30±0.23 | 53-55 | 130-250 |
| Whole pea | 181.50±0.59 | 66-68 | 160-340 |
| Whole pea - LA[1] | 177.35±0.77 | 64-66 | 210-330 |
| Whole pea - St[2] | 173.87±0.92 | 71-73 | 160-270 |
| Whole pea - (LA +St) | 177.50±0.81 | 71-74 | 160-310 |
| Split pea[3] | 175.88±0.68 | 68-73 | 150-260 |
| Split pea - LA[1] | 174.86±0.64 | 69-72 | 170-300 |
| Split pea - St[2] | 176.70±0.76 | 72-74 | 130-300 |
| Split Pea - (LA + St) | 180.03±0.92 | 73-77 | 130-300 |

[1]Leavening agent (LA): sodium bicarbonate added at 0.4% (w/w).
[2]Starch (St): Hylon V added at 20% (w/w).
[3]Split pea: Split and decorticated dry pea.

**Example 3**

**Acceptability of extrudates**

Decorticated Red Chief lentils (Lens culinaris L.) were obtained from Moscow Idaho Seed Co., Moscow, ID., were homogenized and ground to a fine flour in a Pin Mill. The lentil flour was then formulated according to Table 20.

**Table 20. Lentil based formulations containing different texture modifiers**

| # of Runs | Lentils | [1]A P. | [2]W. B. | [3]PB800 | [4]AP40 | Salt | Sugar | [5]Thermolec | [6]Yelkin | [7]Dimo | [8]Pano | Total lbs/batch ("as is") |
|---|---|---|---|---|---|---|---|---|---|---|---|---|
| 1 | 100 | 0 | 0 | 0 | 0 | 0 | 0 | 0 | 0 | 0 | 0 | 100 |
| 2 | 66.75 | 5 | 0 | 20 | 0 | 3 | 5 | 0.25 | 0 | 0 | 0 | 100 |
| 3 | 66.5 | 5 | 0 | 20 | 0 | 3 | 5 | 0.5 | 0 | 0 | 0 | 100 |
| 4 | 66.25 | 5 | 0 | 20 | 0 | 3 | 5 | 0.75 | 0 | 0 | 0 | 100 |
| 5 | 66 | 5 | 0 | 20 | 0 | 3 | 5 | 1 | 0 | 0 | 0 | 100 |
| 6 | 66.75 | 5 | 0 | 20 | 0 | 3 | 5 | 0 | 0.25 | 0 | 0 | 100 |
| 7 | 66.5 | 5 | 0 | 20 | 0 | 3 | 5 | 0 | 0.5 | 0 | 0 | 100 |
| 8 | 66.25 | 5 | 0 | 20 | 0 | 3 | 5 | 0 | 0.75 | 0 | 0 | 100 |
| 9 | 66 | 5 | 0 | 20 | 0 | 3 | 5 | 0 | 1 | 0 | 0 | 100 |
| 10 | 66.75 | 5 | 0 | 20 | 0 | 3 | 5 | 0 | 0 | 0.25 | 0 | 100 |
| 11 | 66.5 | 5 | 0 | 20 | 0 | 3 | 5 | 0 | 0 | 0.5 | 0 | 100 |
| 12 | 66.25 | 5 | 0 | 20 | 0 | 3 | 5 | 0 | 0 | 0.75 | 0 | 100 |
| 13 | 66 | 5 | 0 | 20 | 0 | 3 | 5 | 0 | 0 | 1 | 0 | 100 |
| 14 | 66.75 | 5 | 0 | 20 | 0 | 3 | 5 | 0 | 0 | 0 | 0.25 | 100 |
| 15 | 66.5 | 5 | 0 | 20 | 0 | 3 | 5 | 0 | 0 | 0 | 0.5 | 100 |
| 16 | 66.25 | 5 | 0 | 20 | 0 | 3 | 5 | 0 | 0 | 0 | 0.75 | 100 |
| 17 | 66 | 5 | 0 | 20 | 0 | 3 | 5 | 0 | 0 | 0 | 1 | 100 |

[1]A. P.: Apple fiber.
[2]W. B.: Wheat bran.
[3]PB800: PenBind 800 modified potato starch
[4]AP40: PenPlus 40 pregelatinized potato starch
[5]Thermolec: Thermolec lecithin.
[6]Yelkin: Yelkin TS lecithin.
[7]Dimo: Dimodan PH 100 K-A
[8]Pano: Panodan FDP K

A Clextral Evolum HT 32H twin-screw extrusion system (Clextral-Bivis, Firminy Cedex, France) was used in this study. The heating profiles for the six barrel sections of the extruder were 15, 80, 100, 120, 140, and 160°C, respectively. Flours were fed into the extruder feed port at a rate of 25 kg/h and the extruder was run at two screw speeds of 500 and 700 rpm. Water was added into the extruder through a variable piston pump (Model P5-120, Bran and Luebbe, Wheeling, IL) to bring the moisture contend of the feed under extrusion to 17% (wwb). When the processing conditions of torque and temperature were at steady state the extrudates, coming out of 2 circular dies 3 mm in diameter, were collected for 5 min.

Result of previous sensory evaluation of legume extrudates indicated that the legume based snacks and breakfast cereal type products have a sticky mouth feeling. This was mainly attributed to their higher protein content.

Therefore texture modifiers (emulsifiers) were used to minimize the unpleasant "sticky" sensory effect in the extrudate and improve their texture and acceptability. The texture modifiers used in the study were Dimodan PH 100 K-A and Panodan FDP K (Danisco Co., Richmond, IL) in powder form; Yelkin TS Lecithin and Thermolec Lecithin (ADM Co., Decatur, I11) in liquid form. Each of the emulsifiers was used at the following concentrations: 0.25. 0.50, 0.75 and 1.00%.

Preliminary sensory evaluation: Expansion ratio is a leading parameter to consider in the fabrication of expanded snacks of breakfast cereal type products. Therefore, to facilitate the sensory evaluation of the samples, the 32 generated samples were pre-sorted based on their maximum expansion ratio. Sixteen samples were selected, among the 32 generated samples. The expansion ratio of the selected 16 samples varied from 7.99 to 13.60. The first stage of the sensory evaluation consisted in evaluating the pre-sorted 16 samples for expansion, texture, flavor and overall acceptability of the extrudates. The goal of the first evaluation stage was to determine the 4 most acceptable extrudates among the tested emulsifiers. Lentil extrudates, in the form of rods, were cut into 1.25" length, placed in a pre-coded tray and then evaluated by 19 untrained judges using a score from 1=worst to 5= best.

Table 21 shows the 4 selected lentil based extrudates selected from the first sensory evaluation stage. Results demonstrated that the most acceptable extrudate was that containing Dimodan PH 100 K-A at a concentration of 0.75% and run at 500 rpm. The second and third most acceptable extrudates were those containing Yelkin TS Lecithin at a concentration of 0.75% and run at 500 rpm and Dimodan PH 100 K-A at a concentration of 0.25% and run at 500 rpm, respectively. The least acceptable extrudate of this group was that containing Yelkin TS Lecithin at a concentration of 0.25% and run at 700 rpm. The range of expansion ratio of the selected samples range from 8.75 to 10.24. It was important to notice that when the expansion ratio was in this range, the selection of the best extrudate was mainly due to the type and concentration of the tested emulsifiers.

**Table 21. Selected lentil based extrudates first sensory evaluation stage**

| Texture Modifier | ER | TM (%) | RPM | Sensory Score |
|---|---|---|---|---|
| Yelkin | 8.75 | 0.25 | 700 | 218 |
| Dimodan-100 | 10.24 | 0.25 | 500 | 221 |
| Yelkin | 9.92 | 0.75 | 500 | 238 |
| Dimodan-100 | 9.25 | 0.75 | 500 | 245 |
| ER: expansion ratio of extrudate. TM (%): concentration of texture modifiers expressed in percentage in the lentil formulation. RPM: extruder screw speed in revolution per minutes. | | | | |

Based on the result of the first sensory evaluation stage, the 4 selected best samples were further evaluated for a second sensory evaluation stage to select the most acceptable extrudate's containing emulsifier. The sensory evaluation protocol was the same used in the first sensory evaluation stage.

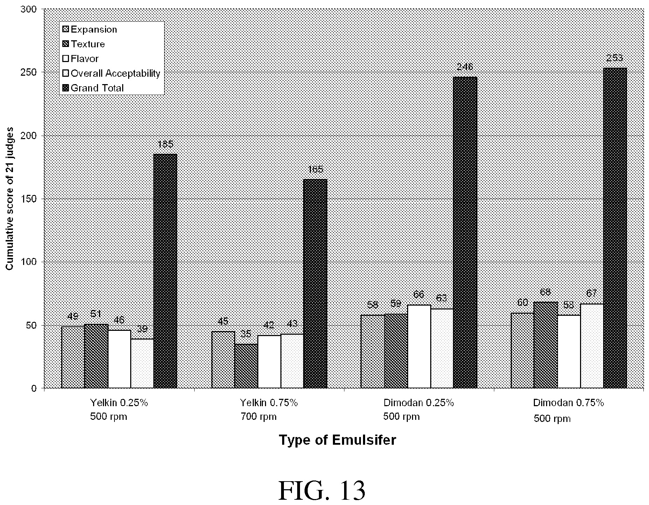

Results of the second sensory evaluation stage demonstrated that the most acceptable extrudate was that containing Dimodan PH 100 K-A at a concentration of 0.75% and run at 500 rpm. The second and third most acceptable extrudates were those containing Dimodan PH 100 K-A at a concentration of 0.25% and run at 500 rpm and Yelkin TS Lecithin at a concentration of 0.25% and run at 700 rpm, respectively. The least acceptable extrudate of this group was that containing Yelkin TS Lecithin at a concentration of 0.75% and run at 500 rpm (Figure 13). The obtained result confirmed what it was found in the first sensory evaluation stage by selecting again the extrudate containing Dimodan PH 100 K-A at a concentration of 0.25% and run at 500 rpm as the most acceptable one (Table 21). However, the extrudate containing Yelkin TS Lecithin at a concentration of 0.25% and run at 700 rpm, which ranked 2nd best on the first sensory evaluation stage, was considered the least acceptable extrudate on the second sensory evaluation stage. Since the sensory evaluation was done with untrained judges, the first stage may have allowed them to gain some experience which was applied in the second stage of the sensory evaluation. Additionally, the second sensory evaluation stage contained only 4 samples vs 16 evaluated in the first stage. This reduced number of samples may have allowed them also more time to evaluate the extrudated. Therefore, we considered the result of the second sensory evaluation stage a more stringent and reliable one.

The most acceptable lentil based extrudate from the second sensory evaluation stage containing Dimodan PH 100 K-A at a concentration of 0.25% and run at 500 rpm, was produced in large quantities to be evaluated by large number of potential commercial consumers at a national food festival.

Toasting of extrudates: Toasting operation removes additional moisture from the extrudate, which promote a more crunchy texture to the product. Also, it facilitates the absorption of oil and flavors by the extrudate during the coating process.

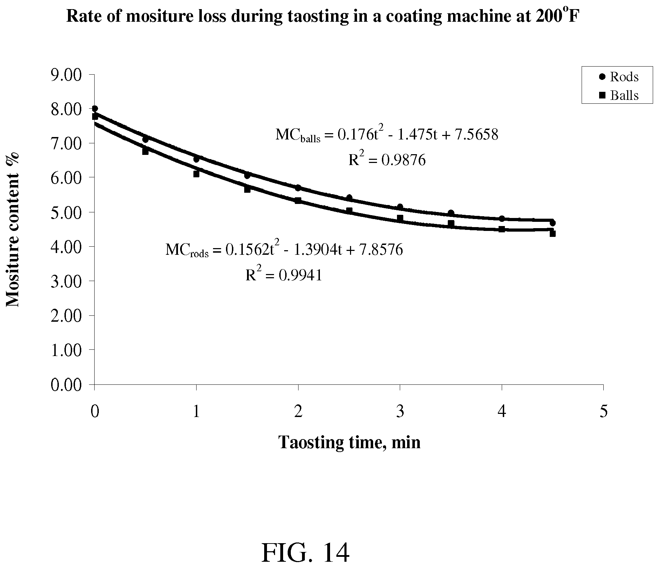

In previous studies, we determined that the coating is done more effectively if the extrudate is toasted at 93°C to 121°C (200 to 250°F). In this study, the toasting of lentil based extrudates was conducted in the rotary drum of a coating machine at 93°C (200°F) for 5 minutes. It was found that the extrudate lost about 2 percent moisture during the toasting operation (Fig. 14). Extrudates were produced in the form of rod and balls as snacks and as breakfast cereal type products, respectively. Additionally, the snack type extrudates were coated with Classic Barbeque (CBQ), Sweet and Bold Barbeque and Cheese and the breakfast cereal type extrudates were coated with sugar for taste.

REFERENCES

AACC. 1984. Approved Methods of the AACC, 8th Ed., Method 44-15. American Association of Cereal Chemists. Paul, MN.

Alonso, R., Rubio, L.A., Muzquiz, M. and Marzo, F. 2001 The Effect of Extrusion Cooking on Mineral Bioavailability In Pea and Kidney Bean Seed Meals. Animal Feed Sci. Technol. Nov. 2001; 94 (12): 1-13.

Allan G.L. and Booth, M.A. 2004. Effects of extrusion processing on digestibility of peas, lupines, canola meal and soybean meal in silver perch Bidyanus bidyanus (Michell) diets. Aquaculture Res. 35: 981-991.

Anderson, R.A., Conway, H.F., Pfeifer, V.F., and Griffin, E.L. Jr. 1969. Gelatinization of Corn Grits by Roll-and Extrusion-Cooking. Cereal Sci. Today. 14 (1): 4-7, 11-12.

Augustin, J. and Klein, B. P. 1989. Nutrient Composition of raw, cooked, canned, and sprouted legumes. In: Legumes: chemistry, technology, and human nutrition. pp. 187-217. Ed. Ruth H. Mathews, Marcel Dekker, Inc. NY.

Balandran-Quintana, R.R., Barbosa-Canovas, G.V., Zezueta-Morales J.J., Anzaldua-Morales, A. and Quintero-Ramos, A. 1998. Functional and nutritional properties of extruded whole pinto bean meal (Phaseolus vulgaris L.) J. Food Sci. 63 (1): 113-116

Berrios, J. De J., Delilah F. Wood, D.F., Whitehand, L. and Pan, J. 2004. Effect of sodium bicarbonate on the microstructure, expansion and color of extruded black beans. J. Food Processing and Preservation. 28: 321-335.

Berrios, J.De J.; Swanson, B.G.; Cheong, W.A. 1998. Structural Characteristics of Stored Black Beans (Phaseolus vulgaris L.). Scanning, J. Scanning Microscopies. 20: 410-417.

Berrios, J.De J.; Swanson, B.G.; Cheong, W.A. 1999. Physico-Chemical Characterization of Stored Black Beans (Phaseolus vulgaris L.). Food Res. nternational. 32: 669-676.

Bhattacharya, S., Chakraborty, P., Chattoraj, D.K. and Mukherjee, S. 1997. Physico-chemical characteristics of extruded snacks prepared from rice (Oryza sativa L.) and chickpea (Cicer arietinum L.) by single screw extrusion. J. Food Sci. Technol-Mysore, 34 (4): 320-323.

Bhattacharya, S. 1997. Twin screw extrusion of rice green gram blend: Extrusion and extrudate characteristics. J. Food Engineering. 32 (2): 83-99.

Björck, I. and Asp, N.-G. 1983. The effects of extrusion cooking on nutritional value - A literature review. J. Food Eng. 2: 281-308.

Björck, I.; Nyman, M.; Asp, N-G. 1984. Extrusion Cooking and Dietary Fiber: Effects of Dietary Fiber Content on Degradation in the Rat Intestinal Tract. Cereal Chem. 61: 174-179.

Borlongan, I.F., Eusebio, P.S. and Welsh, T. 2003. Potential of feed pea (Pisum sativum) meal as a protein source in practical diets for milkfish (Chanos chanos Forsskal). 2003. Aquaculture. 225: 89-98.

Bressani, R.; Elias, L.G.; Valiente, A.T. 1963. Effect of Cooking and Amino Acid Supplementation on the Nutritive Value of Black Beans (Phaseolus vulgaris L.). British J. Nutr. 17: 69-78.

Bressani, R. 1985. Protein Quality and Nutritional Value of Beans; Research Highlights; Michigan State University Bean/Cowpea CRSP, 2: 1-4.

Carrillo, J.M., Moreno, C.R., Rodelo, E.A., Trejo, A.C. and Escobedo, R.M. 2000. Physicochemical and Nutritional Characteristics of extruded flours from fresh and hardened chickpeas (Cicer arietinum L) Lebensm.-Wiss. Technol. 33 (2): 117-123.

Chen, J., Serafin, F.L., Pandya, R.N. and Daun, H. 1991. Effects of extrusion conditions on sensory properties of corn meal extrudates. J. Food Sci. 56 (1): 84-89.

Chinnaswamy, R., Hanna, M.A., and Zobel, H.F. 1989. Microstructral, physiochemical, and macromlecular changes in extrusion-cooked and retrograded corn starch. Cereal Foods World 34: 415-422.

Conway, H. F. 1971. Extrusion cooking of cereals and soybeans. Food Prod. Dev. 5: 14-17, 27-29.

Czarnecki, Z., Gujska, E. and Khan, K. 1993. Extrusion of pinto bean high protein fraction pretreated with papain and cellulase enzymes. J. Food Sci. 58 (2): 395-398.

Della Valle, G., Boché, Y., Colonna, P. , Vergnes, B. 1995. The extrusion behaviour of potato starch, Carbohydrate Polymers, 28(3):255-264

Della Valle, G., Vergnes, B., Colonna, P., and Patria, A. 1997. Relations between Rheological Properties of Molten Starches and their Expansion Behaviour in Extrusion. J Food Eng. 31(3): 277-296.

Delort-Laval, J. and Mercier, C. 1976. Selection of treatments and study of their influence on the carbohydrate fraction of wheat, barley and maize. Am. Zootechnol. 25: 3-12.

Edwards, R.H., Becker, R., Mossman, A.P., Gray, G.M., and Whitehand, L.C. 1994. Twin-screw extrusion cooking of small white beans (phaseolus vulgaris). LWT 27(5): 472-481.

Eerlingen, R.C., Jacobs, H., Block, K. and Delcour J. A. 1997. Effects of hydrothermal treatments on the rheological properties of potato starch, Carbohydrate Research, 297(4):347-356

Fichtali, J. and van de Voort, R.F. 1989. Fundamental and Practical Aspects of Twin Screw Extrusion. Cereal Foods World. 34 (11), 921-929

Gonzalez, Z. and Perez, E. 2002. Evaluation of Lentil Starches Modified by Microwave Irradiation and Extrusion Cooking. Food Res. International. 35 (5): 415-420.

Guha, M., Ali, Z. S., Bhattacharya, S.1997. Twin-screw Extrusion of Rice Flour Without a Die: Effect of Barrel Temperature and Screw Speed on Extrusion and Extrudate Characteristics, Journal of Food Engineering, 32(3): 251-267

Gujral, S. H., Singh, N., Singh, B. 2003. Application of image analysis to measure screw speed influence on physical properties of corn and wheat extrudates, Journal of Food Engineering, 57(2): 145-152

Gujska, E. and Khan, K. 1990. Effect of temperature on properties of extrudates from high starch fractions of navy, pinto and garbanzo beans. J. Food Sci. 55 (2): 466-469.

Hardacre, A.K., Clark, S.M., Riviere, S., Monro, J.A. and Hawkins, A.J. 2006. Some textural, sensory and nutritional properties of expanded snack food wafers made from corn, lentil and other ingredients. J. Texture Studies. 37: 94-111.

Harper, J.M. 1981. Extrusion of foods, Vol. 2, CRC Inc., Boca Raton, F1.

Harper, J.M. 1986. Extrusion texturization of food. Food Technol. 40 (3): 70-76.

Hsu, H.W., Vavak, D.L., Satterlee, L.D. and Miller, G.A. 1977. A multienzyme technique for estimating protein digestibility. J. Food Sci. 42 (5): 269-1273.

Ilo, S., Liu, Y., and Berghofer, E. 1999.Extrusion cooking of rice flour and amaranth blends. LWT 32(2): 79-88.

Food and Agriculture Organization of the United Nations (FAO). 1991. FAO Production Yearbook 45. FAO, Rome.

Ilo, S., Liu, Y. and Berghofer, E. 1999. Extrusion cooking of rice flour and amaranth blends. Lebensm.-Wiss. Technol. 32 (2): 79-88.

Ilo, S., Tomschik, U., Berghofer, E., and Mundigler, N. 1996. The effect of extrusion operating conditions on the apparent viscosity and the properties of extrudates in twin-screw extrusion cooking of maize grits. LWT 29(7):593-598.

Jenab, M.; Thompson, L. 2002. Role of Phytic Acid in Cancer and other Diseases. In Food Phytates; Reddy, N. R., Sathe. S. K., Eds.; CRC Press.: Boca Raton, F1 280-282.

Jenkins, D.J.A.; Wolever, T.M.S.; Kalmusky, J. 1987. Low-Glycemic-Index Diet in hyperlipidemia: Use of Traditional Starchy Foods. Am. J. Clin. Nutr. 46: 66-71.

Jenkins, D.J.A.; Wolever, T.M.S.; Taylor, R.H.; Barker, H. 1980. Exceptionally Low Blood Glucose Response to Dried Beans: Comparison with other Carbohydrate Foods. British Med. J. 281: 578-580.

Jin Z, Hsieh F and Huff HE. 1995. Effects of soy fiber, salt, sugar and screw speed on physical properties and microstructure of corn meal extrudate. J. Cereal Sci. 22 (2):185-194.

Kereliuk, G. R. and Sosulski, F. W. 1996.Comparison of Starch from Flint Corn with that from Dent Corn and Potato, LWT. 29(4):349-356

Laufer-Marquez, U.M. and Lajolo, F.M. 1991. In vivo digestibility of bean (Phaseolus vulgaris L.) proteins: The role of endogenous protein. J. Agric. Food Chem. 39 (7):1211-1215.

Leathwood, P.; Pollet, P. 1988. Effects of Slow Release Carbohydrates in the Form of Fean Flakes on the Evolution of Hunger and Satiety in Man. Appetite. 10: 1-11.

Li, M. and Lee, T.C. 2000. Effect of extrusion temperature on the solubility and molecular weight of lentil bean flour proteins containing low cysteine residues. J. Agric. Food Chem. 48 (3): 880-884.

Liu, Y.; Hsieh, F.; Heymann, H., H.E.Huff: Journal of food science. 2000. Effect of process conditions on the physical and sensory properties of extruded oat-corn puff. 65(7): 1253-1259.

Maga, J.A. 1978. Cis-Trans fatty acid ratios as influenced by product and temperature of extrusion cooking. Lebensm. Wiss Technol. 11:192-194.

Matthey, F.P. and Hanna, M.A. 1997. Physical and functional properties of twin screw extruded whey protein concentrate-corn starch blends. Lebensm.-Wiss. Technol. 30 (4): 359-366.

Mercier C, C., Linko, P. and Harper, J. M. 1989. Extrusion Cooking of Starch and Starchy Products. In Ch. 9. In Extrusion Cooking. C.C. Mercier, P. Linko, and J.M Harper (ED.), p. 263. AACC, Inc., St. Paul, MN.

Morrison, K.J., and F.J. Muehlbauer. 1986. 'Brewer' lentils. U.S. Department of Agriculture, Agricultural Research Service, Technical Bulletin No. 1408.

Morrow, B. 1991. Rebirth of legumes. Food Technol. 45 (9):96,121.

Muehlbauer, F.J., Summerfield, R.J., Kaiser, W.J., Clement, S.L., Boerboom, C.M., Welsh-Maddux, M.M. and Short, R.W. 1998. Principles and Practice of Lentil Production. U.S. Department of Agriculture, Agricultural Research Service, ARS 141.

Nierle, W., El Bayá, A.W., Seiler, K., Fretzdorff, B. and Wolff, J. 1980. Veränderungen der Getreideinhaltsstoffe während der Extrusion mit einem Doppelschneckenextruder, Getreide Mehl Brot. 34:73-76.

Onwulata, C.I., Konstance, R.P., Smith, P.W., and Holsinger, V.H. 2001a. Co-extrusion of dietary fiber and milk proteins in expanded corn products. LWT 34(7):424-429.

Onwulata, C.I., Smith, P.W., Konstance, R.P., and Holsinger, V.H. 2001b. Incorporation of whey products in extruded corn, potato or rice snacks. Food Research International 34(8): 679-687.

Owusu-Ansah, J., van de Voort, F.R., and Stanley, D.W. 1984. Texture and microstructure changes in corn starch as a function of extrusion variables. Can. Inst. J. Food Sci. Technol. 17(2): 65-70.

Padmanabhan, M., and Bhattacharya, M. 1989. Extrudate expansion during extrusion cooking of foods. Cereal Foods World 34(11): 945-949.

Pelembe, L.A.M., Erasmus, C., and Taylor, J.R.N. 2002. Development of a protein-rich composite sorghum-cowpea instant porridge by extrusion cooking process. LWT 35(2): 120-127.

Phillips, R.D., Chhinnan, M.S. and Kennedy, M.B. 1984. Effect of feed moisture and barrel temperature on physical properties of extruded cowpea meal. J. Food Science 49: 916-921.

Phillips, R.D. 1988. Effect of extrusion cooking on the nutritional quality of plant proteins. In: Protein Quality and the Effects of Processing. R.D. Phillips and J. W. Filnley, Eds., Marcel Dekker, New York.

Poltronieri, F., Areas, J.A.G. and Colli, C. 2000. Extrusion and Iron Bioavailability In Chickpea (Cicer arietinum L.). Food Chem. 70 (2): 175-180.

Rajawat, P., Kushwah, A. and Kushwah, H.S. 2000. Effect of Extrusion Cooking on Some Functional Properties of Faba Bean (Vicia faba L.). J. Food Sci. Technol. Nov-Dec. 2000; 37 (6): 667-670.

Roche Vitamin newsletter. 1998. Vitamin nutrition research newsletter. 5 (2): 1-7. HHN0720-20.

Seker, M., Sadikoglu, H., Ozdemir, M. and Hanna, M. A.. 2003. Phosphorus binding to starch during extrusion in both single- and twin-screw extruders with and without a mixing element, Journal of Food Engineering, 59(4):355-360

Singh, J. and Singh, N. 2003. Studies on the morphological and rheological properties of granular cold water soluble corn and potato starches, Food Hydrocolloids, 17(1): 63-72

Singh, J., Singh N.and Saxena, S. K. 2002. Effect of fatty acids on the rheological properties of corn and potato starch, Journal of Food Engineering, 52(1): 9-16

Singh, V. and Ali, S. Z.. 2000. Acid degradation of starch. The effect of acid and starch type, Carbohydrate Polymers, 41(2): 191-195

Srihara, P. and Alexander, J.C. 1984. Effect of heat treatment on nutritive quality of plant protein blends. Can. Inst. Food Sci. Technol. J. 2: 237-240.

Stanley, D.W. 1989. Protein reactions during extrusion processing. In: Extrussion Cooking. C. Mercier, P. Linko, and J. M. Harper, Eds., American Association of Cereal Chemists, St. Paul, MN, pp. 321-341.

Stauffer, C.E. 1999. Fats and Oils. Practical guides for the food industry. Eagan Press Handbook Series., Chapter 5. Eagan Press, St. Paul, MN.

Takeoka, G.R.; Dao, L.T.; Full, G.H.; Wong, R.Y.; Harden, L.A.; Edwards, R. H.; Berrios, J. De J. 1997. Characterization of Black Bean (Phaseolus vulgaris L.) Anthocyanins. J. Agric. Food Chem. 45: 3395-3400.

Van Den Einde, R.M., Van Der Goot, A.J., Boom, R.M. 2003. Understanding Molecular Weight Reduction Of Starch During Heating-Shearing Processes J Food Sci. 68(8).2396-404

X-RITE. 1993. A guide to understanding color communication. X-Rite Form L10-001 (Rev.8-90). P/N918-801. X-Rite Inc. Grandville, MI.

**Claims**

1. A food product for use in the treatment of obesity or for use in decreasing retroperitoneal adipose accumulation, wherein said food product comprises extruded legumes having uniform expansion ratio values of 6 or greater fortified with yeast autolysate.

2. The food product for the use according to claim 1, wherein the legume is selected from the group consisting of pulses, soybeans, lupins, groundnuts and clover.

3. The food product for the use according to claim 1 or claim 2, wherein the yeast autolysate is present from 2% to 20% by weight of said food product.

4. The food product for the use according to claim 3, wherein the yeast autolysate is present from 12% to 20% by weight of said food product.

5. The food product for the use according to any one of claims 1 to 4, wherein the food product has a water activity (Aw) of less than or equal to 0.4.

6. The food product for the use according to any one of claims 1 to 5, wherein the food product further comprises chromium derived from chromium yeast.

7. The food product for the use according to claim 6, wherein the chromium is present from 1 - 30 ppm.

**Patentansprüche**

1. Nahrungsmittelprodukt zur Verwendung bei der Behandlung von Fettleibigkeit oder zur Verwendung zur Reduzierung der retroperitonialen Fettgewebeakkumulation, worin das Nahrungsmittelprodukt extrudierte Leguminosen mit gleichförmigen Expansionsverhältniswerten von 6 oder höher aufweist und mit Hefeautolysat angereichert ist.

2. Nahrungsmittelprodukt zur Verwendung gemäß Anspruch 1, worin die Leguminose ausgewählt ist aus der Gruppe bestehend aus Hülsenfrüchten, Sojabohnen, Lupinen, Erdnüssen und Klee.

3. Nahrungsmittelprodukt zur Verwendung gemäß Anspruch 1 oder Anspruch 2, worin das Hefeautolysat in einer Menge von 2 bis 20 Gew.-% des Nahrungsmittelprodukts vorhanden ist.

4. Nahrungsmittelprodukt zur Verwendung gemäß Anspruch 3, worin das Hefeautolysat in einer Menge von 12 bis 20 Gew.-% des Nahrungsmittelprodukts vorhanden ist.

5. Nahrungsmittelprodukt zur Verwendung gemäß einem der Ansprüche 1 bis 4, worin das Nahrungsmittelprodukt eine Wasseraktivität (Aw) von weniger als oder gleich 0.4 aufweist.

6. Nahrungsmittelprodukt zur Verwendung gemäß einem der Ansprüche 1 bis 5, worin das Nahrungsmittelprodukt ferner Chrom umfasst, welches aus Chromhefe stammt.

**7.** Nahrungsmittelprodukt zur Verwendung gemäß Anspruch 6, worin das Chrom in einer Menge von 1 - 30 ppm enthalten ist.

**Revendications**

**1.** Produit alimentaire pour une utilisation dans le traitement de l'obésité ou pour une utilisation dans la réduction de l'accumulation d'adipose rétropéritonéale, dans lequel ledit produit alimentaire comprend des légumes extrudés ayant des valeurs de rapport d'expansion uniforme de 6 ou plus fortifiés avec un autolysat de levure.

**2.** Produit alimentaire pour une utilisation selon la revendication 1, dans lequel le légume est choisi dans le groupe constitué des légumineuses, des graines de soja, des lupins, des arachides et du trèfle.

**3.** Produit alimentaire pour une utilisation selon la revendication 1 ou la revendication 2, dans lequel l'autolysat de levure est présent de 2 % à 20 % en poids dudit produit alimentaire.

**4.** Produit alimentaire pour une utilisation selon la revendication 3, dans lequel l'autolysat de levure est présent de 12 % à 20 % en poids dudit produit alimentaire.

**5.** Produit alimentaire pour une utilisation selon l'une quelconque des revendications 1 à 4, dans lequel le produit alimentaire a une activité de l'eau (Aw) inférieure ou égale à 0,4.

**6.** Produit alimentaire pour une utilisation selon l'une quelconque des revendications 1 à 5, dans lequel le produit alimentaire comprend en outre du chrome dérivé de levure de chrome.

**7.** Produit alimentaire pour une utilisation selon la revendication 6, dans lequel le chrome est présent de 1 à 30 ppm.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

Rate of mositure loss during taosting in a coating machine at 200°F

$MC_{balls} = 0.176t^2 - 1.475t + 7.5658$

$R^2 = 0.9876$

$MC_{rods} = 0.1562t^2 - 1.3904t + 7.8576$

$R^2 = 0.9941$

● Rods
■ Balls

Mositure content %

Taosting time, min

FIG. 14

FIG. 15

EP 2 787 840 B1

FIG. 16

43

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008145483 A

**Non-patent literature cited in the description**

- *Nut. Res.,* vol. 30 (2), 141-150
- Approved Methods of the AACC. **PAUL, MN.** AACC. American Association of Cereal Chemists, 1984
- **ALONSO, R. ; RUBIO, L.A. ; MUZQUIZ, M. ; MARZO, F.** 2001 The Effect of Extrusion Cooking on Mineral Bioavailability In Pea and Kidney Bean Seed Meals. *Animal Feed Sci. Technol.,* November 2001, vol. 94 (12), 1-13
- **ALLAN G.L. ; BOOTH, M.A.** Effects of extrusion processing on digestibility of peas, lupines, canola meal and soybean meal in silver perch Bidyanus bidyanus (Michell) diets. *Aquaculture Res.,* 2004, vol. 35, 981-991
- **ANDERSON, R.A. ; CONWAY, H.F. ; PFEIFER, V.F. ; GRIFFIN, E.L. JR.** Gelatinization of Corn Grits by Roll-and Extrusion-Cooking. *Cereal Sci. Today.,* 1969, vol. 14 (1), 4-7
- Nutrient Composition of raw, cooked, canned, and sprouted legumes. **AUGUSTIN, J. ; KLEIN, B. P.** Legumes: chemistry, technology, and human nutrition. Marcel Dekker, Inc, 1989, 187-217
- **BALANDRAN-QUINTANA, R.R. ; BARBOSA-CANOVAS, G.V. ; ZEZUETA-MORALES J.J. ; ANZALDUA-MORALES, A. ; QUINTERO-RAMOS, A.** Functional and nutritional properties of extruded whole pinto bean meal (Phaseolus vulgaris L.). *J. Food Sci.,* 1998, vol. 63 (1), 113-116
- **BERRIOS, J. DE J. ; DELILAH F. WOOD ; D.F., WHITEHAND, L. ; PAN, J.** Effect of sodium bicarbonate on the microstructure, expansion and color of extruded black beans. *J. Food Processing and Preservation,* 2004, vol. 28, 321-335
- **BERRIOS, J.DE J. ; SWANSON, B.G. ; CHEONG, W.A.** Structural Characteristics of Stored Black Beans (Phaseolus vulgaris L.). *Scanning, J. Scanning Microscopies,* 1998, vol. 20, 410-417
- **BERRIOS, J.DE J. ; SWANSON, B.G. ; CHEONG, W.A.** Physico-Chemical Characterization of Stored Black Bean (Phaseolus vulgaris L.). *Food Res. nternational,* 1999, vol. 32, 669-676

- **BHATTACHARYA, S. ; CHAKRABORTY, P. ; CHATTORAJ, D.K. ; MUKHERJEE, S.** Physico-chemical characteristics of extruded snacks prepared from rice (Oryza sativa L.) and chickpea (Cicer arietinum L.) by single screw extrusion. *J. Food Sci. Technol-Mysore,* 1997, vol. 34 (4), 320-323
- **BHATTACHARYA, S.** Twin screw extrusion of rice green gram blend: Extrusion and extrudate characteristics. *J. Food Engineering,* 1997, vol. 32 (2), 83-99
- **BJÖRCK, I. ; ASP, N.-G.** The effects of extrusion cooking on nutritional value - A literature review. *J. Food Eng.,* 1983, vol. 2, 281-308
- **BJÖRCK, I. ; NYMAN, M. ; ASP, N-G.** Extrusion Cooking and Dietary Fiber: Effects of Dietary Fiber Content on Degradation in the Rat Intestinal Tract. *Cereal Chem.,* 1984, vol. 61, 174-179
- **BORLONGAN, I.F. ; EUSEBIO, P.S. ; WELSH, T.** Potential of feed pea (Pisum sativum) meal as a protein source in practical diets for milkfish (Chanos chanos Forsskal). *Aquaculture,* 2003, vol. 225, 89-98
- **BRESSANI, R. ; ELIAS, L.G. ; VALIENTE, A.T.** Effect of Cooking and Amino Acid Supplementation on the Nutritive Value of Black Beans (Phaseolus vulgaris L.). *British J. Nutr.,* 1963, vol. 17, 69-78
- Protein Quality and Nutritional Value of Beans. **BRESSANI, R.** Research Highlights. Michigan State University Bean/Cowpea CRSP, 1985, vol. 2, 1-4
- **CARRILLO, J.M. ; MORENO, C.R. ; RODELO, E.A. ; TREJO, A.C. ; ESCOBEDO, R.M.** Physicochemical and Nutritional Characteristics of extruded flours from fresh and hardened chickpeas (Cicer arietinum L) Lebensm.-Wiss. *Technol.,* 2000, vol. 33 (2), 117-123
- **CHEN, J. ; SERAFIN, F.L. ; PANDYA, R.N. ; DAUN, H.** Effects of extrusion conditions on sensory properties of corn meal extrudates. *J. Food Sci.,* 1991, vol. 56 (1), 84-89

- **CHINNASWAMY, R. ; HANNA, M.A. ; ZOBEL, H.F.** Microstructral, physiochemical, and macromlecular changes in extrusion-cooked and retrograded corn starch. *Cereal Foods World,* 1989, vol. 34, 415-422
- **CONWAY, H. F.** Extrusion cooking of cereals and soybeans. *Food Prod. Dev.,* 1971, vol. 5, 14-17, 27-29
- **CZARNECKI, Z. ; GUJSKA, E. ; KHAN, K.** Extrusion of pinto bean high protein fraction pretreated with papain and cellulase enzymes. *J. Food Sci.,* 1993, vol. 58 (2), 395-398
- **DELLA VALLE, G. ; BOCHÉ, Y. ; COLONNA, P. ; VERGNES, B.** The extrusion behaviour of potato starch. *Carbohydrate Polymers,* 1995, vol. 28 (3), 255-264
- **DELLA VALLE, G. ; VERGNES, B. ; COLONNA, P. ; PATRIA, A.** Relations between Rheological Properties of Molten Starches and their Expansion Behaviour in Extrusion. *J Food Eng.,* 1997, vol. 31 (3), 277-296
- **DELORT-LAVAL, J. ; MERCIER, C.** Selection of treatments and study of their influence on the carbohydrate fraction of wheat, barley and maize. *Am. Zootechnol.,* 1976, vol. 25, 3-12
- **EDWARDS, R.H. ; BECKER, R. ; MOSSMAN, A.P. ; GRAY, G.M. ; WHITEHAND, L.C.** Twin-screw extrusion cooking of small white beans (phaseolus vulgaris). *LWT,* 1994, vol. 27 (5), 472-481
- **EERLINGEN, R.C. ; JACOBS, H. ; BLOCK, K. ; DELCOUR J. A.** Effects of hydrothermal treatments on the rheological properties of potato starch. *Carbohydrate Research,* 1997, vol. 297 (4), 347-356
- **FICHTALI, J. ; VAN DE VOORT, R.F.** Fundamental and Practical Aspects of Twin Screw Extrusion. *Cereal Foods World.,* 1989, vol. 34 (11), 921-929
- **GONZALEZ, Z. ; PEREZ, E.** Evaluation of Lentil Starches Modified by Microwave Irradiation and Extrusion Cooking. *Food Res. International.,* 2002, vol. 35 (5), 415-420
- **GUHA, M. ; ALI, Z. S. ; BHATTACHARYA, S.** Twin-screw Extrusion of Rice Flour Without a Die: Effect of Barrel Temperature and Screw Speed on Extrusion and Extrudate Characteristics. *Journal of Food Engineering,* 1997, vol. 32 (3), 251-267
- **GUJRAL, S. H. ; SINGH, N. ; SINGH, B.** Application of image analysis to measure screw speed influence on physical properties of corn and wheat extrudates. *Journal of Food Engineering,* 2003, vol. 57 (2), 145-152
- **GUJSKA, E. ; KHAN, K.** Effect of temperature on properties of extrudates from high starch fractions of navy, pinto and garbanzo beans. *J. Food Sci.,* 1990, vol. 55 (2), 466-469
- **HARDACRE, A.K. ; CLARK, S.M. ; RIVIERE, S. ; MONRO, J.A. ; HAWKINS, A.J.** Some textural, sensory and nutritional properties of expanded snack food wafers made from corn, lentil and other ingredients. *J. Texture Studies,* 2006, vol. 37, 94-111
- **HARPER, J.M.** Extrusion of foods. CRC Inc, 1981, vol. 2
- **HARPER, J.M.** Extrusion texturization of food. *Food Technol.,* 1986, vol. 40 (3), 70-76
- **HSU, H.W. ; VAVAK, D.L. ; SATTERLEE, L.D. ; MILLER, G.A.** A multienzyme technique for estimating protein digestibility. *J. Food Sci.,* 1977, vol. 42 (5), 269-1273
- **ILO, S. ; LIU, Y. ; BERGHOFER, E.** Extrusion cooking of rice flour and amaranth blends. *LWT,* 1999, vol. 32 (2), 79-88
- FAO Production Yearbook 45. Food and Agriculture Organization of the United Nations (FAO), 1991
- **ILO, S. ; LIU, Y. ; BERGHOFER, E.** Extrusion cooking of rice flour and amaranth blends. *Lebensm.-Wiss. Technol.,* 1999, vol. 32 (2), 79-88
- **ILO, S. ; TOMSCHIK, U. ; BERGHOFER, E. ; MUNDIGLER, N.** The effect of extrusion operating conditions on the apparent viscosity and the properties of extrudates in twin-screw extrusion cooking of maize grits. *LWT,* 1996, vol. 29 (7), 593-598
- Role of Phytic Acid in Cancer and other Diseases. **JENAB, M. ; THOMPSON, L.** Food Phytates. CRC Press, 2002, 280-282
- **JENKINS, D.J.A. ; WOLEVER, T.M.S. ; KALMUSKY, J.** Low-Glycemic-Index Diet in hyperlipidemia: Use of Traditional Starchy Foods. *Am. J. Clin. Nutr.,* 1987, vol. 46, 66-71
- **JENKINS, D.J.A. ; WOLEVER, T.M.S. ; TAYLOR, R.H. ; BARKER, H.** Exceptionally Low Blood Glucose Response to Dried Beans: Comparison with other Carbohydrate Foods. *British Med. J.,* 1980, vol. 281, 578-580
- **JIN Z ; HSIEH F ; HUFF HE.** Effects of soy fiber, salt, sugar and screw speed on physical properties and microstructure of corn meal extrudate. *J. Cereal Sci.,* 1995, vol. 22 (2), 185-194
- **KERELIUK, G. R. ; SOSULSKI, F. W.** Comparison of Starch from Flint Corn with that from Dent Corn and Potato. *LWT.,* 1996, vol. 29 (4), 349-356
- **LAUFER-MARQUEZ, U.M. ; LAJOLO, F.M.** vivo digestibility of bean (Phaseolus vulgaris L.) proteins: The role of endogenous protein. *J. Agric. Food Chem.,* 1991, vol. 39 (7), 1211-1215
- **LEATHWOOD, P. ; POLLET, P.** Effects of Slow Release Carbohydrates in the Form of Fean Flakes on the Evolution of Hunger and Satiety in Man. *Appetite,* 1988, vol. 10, 1-11
- **LI, M. ; LEE, T.C.** Effect of extrusion temperature on the solubility and molecular weight of lentil bean flour proteins containing low cysteine residues. *J. Agric. Food Chem.,* 2000, vol. 48 (3), 880-884

- **LIU, Y. ; HSIEH, F. ; HEYMANN, H. ; H.E.HUFF.** Effect of process conditions on the physical and sensory properties of extruded oat-corn puff. *Journal of food science,* 2000, vol. 65 (7), 1253-1259
- **MAGA, J.A.** Cis-Trans fatty acid ratios as influenced by product and temperature of extrusion cooking. *Lebensm. Wiss Technol.,* 1978, vol. 11, 192-194
- **MATTHEY, F.P. ; HANNA, M.A.** Physical and functional properties of twin screw extruded whey protein concentrate-corn starch blends. *Lebensm.-Wiss. Technol.,* 1997, vol. 30 (4), 359-366
- Extrusion Cooking of Starch and Starchy Products. **MERCIER C, C. ; LINKO, P. ; HARPER, J. M.** Extrusion Cooking. AACC, Inc, 1989, 263
- **MORRISON, K.J. ; F.J. MUEHLBAUER.** Brewer' lentils. U.S. Department of Agriculture. *Agricultural Research Service,* 1986
- **MORROW, B.** Rebirth of legumes. *Food Technol.,* 1991, vol. 45 (9), 96, , 121
- **MUEHLBAUER, F.J. ; SUMMERFIELD, R.J. ; KAISER, W.J. ; CLEMENT, S.L. ; BOERBOOM, C.M. ; WELSH-MADDUX, M.M. ; SHORT, R.W.** Principles and Practice of Lentil Production. U.S. Department of Agriculture, Agricultural Research Service, ARS, 1998, 141
- **NIERLE, W. ; EL BAYÁ, A.W. ; SEILER, K. ; FRETZDORFF, B. ; WOLFF, J.** Veränderungen der Getreideinhaltsstoffe während der Extrusion mit einem Doppelschneckenextruder. *Getreide Mehl Brot.,* 1980, vol. 34, 73-76
- **ONWULATA, C.I. ; KONSTANCE, R.P. ; SMITH, P.W. ; HOLSINGER, V.H.** Co-extrusion of dietary fiber and milk proteins in expanded corn products. *LWT,* 2001, vol. 34 (7), 424-429
- **ONWULATA, C.I. ; SMITH, P.W. ; KONSTANCE, R.P. ; HOLSINGER, V.H.** Incorporation of whey products in extruded corn, potato or rice snacks. *Food Research International,* 2001, vol. 34 (8), 679-687
- **OWUSU-ANSAH, J. ; VAN DE VOORT, F.R. ; STANLEY, D.W.** Texture and microstructure changes in corn starch as a function of extrusion variables. *Can. Inst. J. Food Sci. Technol.,* 1984, vol. 17 (2), 65-70
- **PADMANABHAN, M. ; BHATTACHARYA, M.** Extrudate expansion during extrusion cooking of foods. *Cereal Foods World,* 1989, vol. 34 (11), 945-949
- **PELEMBE, L.A.M. ; ERASMUS, C. ; TAYLOR, J.R.N.** Development of a protein-rich composite sorghum-cowpea instant porridge by extrusion cooking process. *LWT,* 2002, vol. 35 (2), 120-127
- **PHILLIPS, R.D. ; CHHINNAN, M.S. ; KENNEDY, M.B.** Effect of feed moisture and barrel temperature on physical properties of extruded cowpea meal. *J. Food Science,* 1984, vol. 49, 916-921
- Effect of extrusion cooking on the nutritional quality of plant proteins. **PHILLIPS, R.D.** Protein Quality and the Effects of Processing. Marcel Dekker, 1988
- **POLTRONIERI, F. ; AREAS, J.A.G. ; COLLI, C.** Extrusion and Iron Bioavailability In Chickpea (Cicer arietinum L.). *Food Chem.,* 2000, vol. 70 (2), 175-180
- **RAJAWAT, P. ; KUSHWAH, A. ; KUSHWAH, H.S.** Effect of Extrusion Cooking on Some Functional Properties of Faba Bean (Vicia faba L.). *J. Food Sci. Technol.,* 2000, vol. 37 (6), 667-670
- Roche Vitamin newsletter. *Vitamin nutrition research newsletter,* 1998, vol. 5 (2), 1-7
- **SEKER, M. ; SADIKOGLU, H. ; OZDEMIR, M. ; HANNA, M. A.** Phosphorus binding to starch during extrusion in both single- and twin-screw extruders with and without a mixing element. *Journal of Food Engineering,* 2003, vol. 59 (4), 355-360
- **SINGH, J. ; SINGH, N.** Studies on the morphological and rheological properties of granular cold water soluble corn and potato starches. *Food Hydrocolloids,* 2003, vol. 17 (1), 63-72
- **SINGH, J. ; SINGH N. ; SAXENA, S. K.** Effect of fatty acids on the rheological properties of corn and potato starch. *Journal of Food Engineering,* 2002, vol. 52 (1), 9-16
- **SINGH, V. ; ALI, S. Z.** Acid degradation of starch. The effect of acid and starch type. *Carbohydrate Polymers,* 2000, vol. 41 (2), 191-195
- **SRIHARA, P. ; ALEXANDER, J.C.** Effect of heat treatment on nutritive quality of plant protein blends. *Can. Inst. Food Sci. Technol. J.,* 1984, vol. 2, 237-240
- Protein reactions during extrusion processing. **STANLEY, D.W.** Extrussion Cooking. American Association of Cereal Chemists, 1989, 321-341
- Fats and Oils. Practical guides for the food industry. **STAUFFER, C.E.** Eagan Press Handbook Series. Eagan Press, 1999
- **TAKEOKA, G.R. ; DAO, L.T. ; FULL, G.H. ; WONG, R.Y. ; HARDEN, L.A. ; EDWARDS, R. H. ; BERRIOS, J. DE J.** Characterization of Black Bean (Phaseolus vulgaris L.) Anthocyanins. *J. Agric. Food Chem.,* 1997, vol. 45, 3395-3400
- **VAN DEN EINDE, R.M. ; VAN DER GOOT, A.J. ; BOOM, R.M.** Understanding Molecular Weight Reduction Of Starch During Heating-Shearing Processes. *J Food Sci.,* 2003, vol. 68 (8), 2396-404
- A guide to understanding color communication. **X-RITE.** X-Rite Form L10-001 (Rev.8-90). P/N918-801. X-Rite Inc, 1993